# EUROPÄISCHE PATENTSCHRIFT

(11) **EP 3 655 245 B1**
(45) Veröffentlichungstag und Bekanntmachung des Hinweises auf die Patenterteilung: **15.09.2021**
(21) Anmeldenummer: 18732664.0
(22) Anmeldetag: 15.05.2018
(51) Int. Cl.: B32B 7/12, B32B 15/085, B32B 15/12, B32B 15/18, B32B 15/20, B32B 27/10, B32B 27/18, B32B 27/20, B32B 27/32, B32B 29/00, B32B 3/08, B32B 3/26, B65D 79/02, G06K 19/00, G01N 33/02

(54) **PACKUNGSLAMINAT, ZUSCHNITT, PACKUNGSMANTEL, PACKUNG UND VERPACKUNG MIT ELEKTRISCHEN ELEMENTEN**
PACKING LAMINATE, BLANK, PACKING ENVELOPE, PACKING AND PACKAGING WITH ELECTRICAL ELEMENTS
STRATIFIÉ D'EMBALLAGE, DÉCOUPE, ENVELOPPE D'EMBALLAGE, EMBALLAGE ET CONDITIONNEMENT COMPRENANT DES ÉLÉMENTS ÉLECTRIQUES

(30) Priorität: 18.07.2017 DE 102017116169
(43) Veröffentlichungstag der Anmeldung: 27.05.2020
(73) Patentinhaber: SIG Technology AG, 8212 Neuhausen am Rheinfall (CH)
(72) Erfinder: REISERT, Steffen, 52066 Aachen (DE); SCHLAPPA, Ferdinand, 90491 Nürnberg (DE); MALINDRETOS, Lars, 47804 Krefeld (DE)
(74) Vertreter: Cohausz & Florack
(86) Internationale Anmeldenummer: PCT/EP2018/062469
(87) Internationale Veröffentlichungsnummer: WO 2019/015826

(56) Entgegenhaltungen:
- EP-A1- 1 375 131
- EP-A1- 2 071 496
- EP-A1- 2 390 203
- WO-A1-2014/123463
- WO-A1-2015/126306

## Beschreibung

Der Gegenstand betrifft Zuschnitte, Packungsmäntel, Packungen und Verpackungen, welche mit elektronischen Elementen versehen sind, die insbesondere zur Zustandsüberwachung des verpackten Gutes dienen.

Verpackungen können auf unterschiedliche Weisen und aus verschiedensten Materialien hergestellt werden. Eine weitverbreitete Möglichkeit ihrer Herstellung besteht darin, Verpackungen aus einem Packungslaminat mit einem, auf einem Faserstoff, insbesondere Karton, basierende Trägerschicht aufgebauten Laminat mittels Falt- und Siegelvorgängen herzustellen. Dazu sind im Wesentlichen zwei Verfahren etabliert

Bei einem ersten Verfahren wird aus einem, vorzugsweise von einer Rolle abgewickelten Packungslaminat in dessen Ablaufrichtung (Längsrichtung) ein Schlauch geformt, während die entstehende Packung entlang ihrer Längsnaht meist durch Einbringung eines Siegelstreifens verschlossen wird. In diesen Schlauch wird das durch die entstehende Packung zu schützende Produkt gefüllt und der gefüllte Schlauch an vorgegebenen Stellen quer zur Laufrichtung portionsweise versiegelt und vereinzelt. Aus den so entstandenen halbfertigen Verbundverpackungen ("Kissen") werden durch Falt- und weitere Siegelvorgänge fertige Verbundverpackungen erzeugt

In einem zweiten Verfahren werden aus dem ebenfalls zunächst als Packungslaminat vorliegenden Verpackungsmaterial durch längs- und/oder querschneiden einzelne Zuschnitte hergestellt, aus denen durch Falten und weitere Schritte, wie z.B. Siegeln entlang von Siegelkanten zunächst ein Packungsmantel und schließlich eine Verpackung entsteht. Diese Herstellungsart hat unter anderem den Vorteil, dass die Zuschnitte und Packungsmäntel sehr flach sind und somit platzsparend gestapelt werden können. Auf diese Weise können die Zuschnitte bzw. Packungsmäntel an einem anderen Ort hergestellt werden, als die Faltung, Siegelung und Befüllung der Packungsmäntel erfolgt Auch hier kommen Laminate (=Verbundstoffe) zum Einsatz, die auf einer Trägerschicht aufbauen, die einen Faserstoff, insbesondere Karton aufweisen.

Derartige Verpackungen finden insbesondere in der Lebensmittelindustrie große Verbreitung.

Abhängig davon, ob eine Verbundverpackung einen Schutz über einige Tage bis hin zu wenigen Wochen für ein sogenanntes frisch abgefülltes Produkt bieten soll oder ob es ein "keimfreies" und unter aseptischen Bedingungen abgefülltes Lebensmittel mit wenigstens einem flüssigen Anteil über einen langen Zeitraum bei Umgebungsbedingungen schützen soll, ergeben sich zum Teil sehr unterschiedliche Anforderungen.

Bei einem frisch abgefüllten Produkt, auch wenn es sich um ein sogenanntes "extended-shelflife-Produkt" handelt, ist der Zeitraum, indem der Inhalt der Verbundverpackung zu schützen ist, sehr übersichtlich und beträgt zwischen einigen Tagen bis zu mehreren Wochen. Dafür streuen meist aber die Eigenschaften des Produktes an sich recht stark. So schwankt beispielsweise die das Produkt belastende Keimzahl deutlich von Charge zu Charge des an sich gleichen Produktes, sodass die Anfangsbedingungen nur schwer fassbar sind.

Bei einem "keimfreien" und unter aseptischen Bedingungen abgefüllten Produkt stellen sich dagegen ganz andere Probleme dar. Zwar findet man hier definierte Bedingungen vor, um die die meist zu Zehntausend oder sogar mehreren Zehntausend, während einer Stunde Produktionszeit auf einer einzigen Füllmaschine entstehenden Verbundverpackungen schwanken. Jedoch werden die einzelnen Verbundverpackungen in ihrem weiteren Bestehen dann unterschiedlichen Strapazen ausgesetzt, sodass die für das zu schützende Produkt entstehenden Belastungen, beispielsweise durch Transport- und Lagerbedingungen, über den langen Zeitraum von bis zu einem Jahr oder sogar länger im Einzelfall kaum vorauszusagen sind.

Von einem langanhaltenden Schutz eines Lebensmittels spricht man im Allgemeinen, wenn das Produkt in seiner vollen Qualität über Monate ungekühlt in der Verbundverpackung geschützt werden kann. Das Produkt wird dazu dann im Allgemeinen aseptisch in die entstehende Verbundverpackung gefüllt. Bei H-Milch und Säften kann dann oft davon ausgegangen werden, dass der Inhalt der Verbundverpackung über einem Zeitraum von bis zu einem Jahr, manchmal sogar darüber hinaus haltbar ist.

Um den Verbraucher vor dem Verzehr eines gesundheitsbedenklichen Lebensmittels zu schützen, schreibt der Gesetzgeber trotz der eben beschriebenen, beinahe gegensätzlichen Problematiken einen einheitlichen Lösungsansatz vor, nämlich die Kennzeichnung der Verbundverpackung mit einem so genannten "Mindesthaltbarkeitsdatum". Der Hersteller muss bei diesem Konzept dafür bürgen, dass das in der Verbundverpackung enthaltene Produkt bis zur Erreichung des Mindesthaltbarkeitsdatums gesundheitlich unbedenklich ist und falls auf der Packung angegeben seine Qualität wenigstens den angegebenen Nährwerten entspricht Dies führt automatisch dazu, dass das angegebene Mindesthaltbarkeitsdatum äußerst vorsichtig veranschlagt wird, sodass der Großteil der Verbundverpackungen auch noch -zum Teil deutlich- über den zeitlichen Ablauf des Mindesthaltbarkeitsdatums hinaus ein gesundheitlich völlig unbedenkliches Produkt beinhaltet.

Dennoch führt die Angabe des Mindesthaltbarkeitsdatums dazu, dass der Verbraucher das Produkt heute meist nicht mehr selbstständig testet, sondern nach Ablauf des Mindesthaltbarkeitsdatums im Regelfall die ungeöffnete Verbundverpackung entsorgt Aus der Veröffentlichung EP 2 071 496 A1 ist Verfahren zur Herstellung eines Verpackungsmaterial bekannt, bei dem mittels eines funkauslesbaren Speichers, insbesondere eines RFID Chips, Informationen zu einer Verpackung und einem Produkt gespeichert werden können. Der Speicher wird dabei in einen Verbundstoff eingearbeitet und kann auch in verschiedenen Lagen des Verbundstoffes angeordnet sein. Die Information kann z.B. eine Information zu einem Mindesthaltbarkeitsdatum enthalten. Der Stand der Technik umfasst auch das Dokument EP 2 390 203 A1.

Nachteilig bei dieser aus dem Stand der Technik bekannten Lösung ist jedoch zum Einen, dass die Informationen statisch gespeichert sind und zum Anderen, dass eine Kontaktierung durch die Metallschicht hindurch nicht möglich ist, da die Metallschicht einen elektrischen Kurzschluss der Kontaktierungen bewirken würde.

Es ist daher eine Aufgabe, eine Kommunikation zwischen einer Innenschicht und einer Außenschicht einer Packung zu ermöglichen. Es ist eine weitere Aufgabe den Verbraucher über den tatsächlich vorliegenden Qualitätszustand des Inhalts einer flüssigkeitsdichten Verbundverpackung zu informieren. Im Besonderen soll der Verbraucher eine Information erhalten, ob der Verzehr des Inhalts der Verbundverpackung zum Prüfzeitpunkt unbedenklich ist Zur Lösung dieser Aufgabe wird ein Zuschnitt nach Anspruch 1, ein Packungsmantel nach Anspruch 13, eine Packung nach Anspruch 16 sowie eine Verpackung nach Anspruch 17 vorgeschlagen.

Gegenständlich ist erkannt worden, dass eine Information aus dem Inneren einer Verpackung nach außen nur dann elektrisch übermittelt werden kann, wenn die metallische Barriereschicht durchbrochen wird. Um ein Funktionselement im Inneren der Verpackung elektrisch ansteuern zu können und ggf. Informationen auslesen zu können, ist eine zumindest zweiadrige Verbindung mit einem elektrischen Element auf der Außenseite notwendig. Diese wird gegenständlich durch die Barriereschicht hindurch durch zumindest zwei gegeneinander elektrisch isolierte Leiter bewirkt

Beispielsweise wird ein Packungslaminat (nachfolgend auch als Packstoff bezeichnet) als Bahnware hergestellt. Diese kann als Verbund aus einer oder mehreren dünnen Lagen umfassend zumindest eine Trägerschicht, eine Barriereschicht sowie eine Deckschicht und eine Außenschicht gebildet sein. Die Außenschicht kann ebenfalls als Deckschicht bezeichnet werden. Der genaue Aufbau des Laminats (Verbundstoffs) richtet sich meist im Wesentlichen nach dem gewünschten Maß an Schutz. So weisen Laminate (Verbundstoffe), die zur Herstellung einer Verpackung für einen langanhaltenden Schutz des in ihr zu lagernden zumindest teilweise schüttfähigen, pastösen, und/oder flüssigen Produkts verwendet werden, eine eine zusätzliche Gasbarriere bildende Barriereschicht auf, insbesondere dann, wenn das Produkt oder Teile des Produkts empfindlich auf Luft, insbesondere auf Sauerstoff, reagieren.

Zunächst wird vorgeschlagen, dass der Packstoff aus einem mehrlagigen Laminat gebildet ist. Hierbei kann zumindest eine Trägerschicht mit einer elektrisch leitenden Barriereschicht verbunden sein. Ferner kann zumindest eine Deckschicht vorgesehen sein, die zumindest mit der Barriereschicht verbunden ist

Zur Detektion von Zuständen im Inneren einer nach der Verarbeitung des Packstoffs erhaltenen Verpackung, kann auf einer ersten Seite der Barriereschicht ein Funktionselement angeordnet ist

Ferner kann auf der dem Funktionselement abgewandten zweiten Seite der Barriereschicht ein elektrisches Element angeordnet ist.

Das Funktionselement kann ein elektrisches, elektronisches, chemisches und/oder elektrochemisches Element sein. Das elektrische Element kann elektrische als auch elektronische Komponenten enthalten.

Es ist nun erkannt worden, dass eine elektrische Wirkverbindung zwischen den beiden Elementen durch zumindest zwei elektrische Leiter ermöglicht ist Dabei wird vorgeschlagen, dass das Funktionselement mit dem elektrischen Element über zumindest zwei zueinander isolierte elektrische Leiter verbunden ist Die elektrischen Leiter können im Betrieb zwei voneinander verschiedene elektrische Potentiale haben und somit neben einer Energieversorgung des Funktionselements im Inneren der Packung auch zur Signalübertragung genutzt werden.

Es ist erstmalig möglich, eine elektrische Wirkverbindung zwischen einem Inneren einer Packung und einem äußeren einer Packung bei der Verwendung einer metallischen Barriereschicht bereit zu stellen. Bisher war man davon ausgegangen, dass eine Verletzung der Barriereschicht nachteilig im Hinblick auch die Dichtheit der Verpackung ist Es ist jedoch erkannt worden, dass durch geeignete Anordnung der Leiter, wie nachfolgend noch diskutiert wird, eine solche Befürchtung unbegründet ist

In besonderen Fällen kann auch ein einziger elektrischer Leiter ausreichend sein. Dieser kann gegenüber der Barriereschicht elektrisch isoliert durch die Barriereschicht geführt sein oder durch die Barriereschicht selbst gebildet sein. Auch eine solche Ausgestaltung kann mit allen hier beschriebenen Merkmalen kombinierbar sein. In diesem Zusammenhang sei ausdrücklich darauf hingewiesen, dass die hier beschriebenen Merkmale selbstständig und in Kombination mit anderen hier beschriebenen Merkmalen zweckmäßig eingesetzt werde können.

Gemäß einem Ausführungsbeispiel weist das erste Funktionselement einen Sensor auf. Gemäß einem Ausführungsbeispiel weist das elektrische Element einen Transmitter und/oder eine Antenne auf.

Die Antenne (Antenneneinheit) kann wenigstens eine Leiterbahn, vorzugsweise in Form einer Leiterspirale oder Leiterspule, und/oder Anschlüsse zum Anschließen der Antenneneinheit an eine Chipeinheit umfassen. Auch können die Antenneneinheit und die Chipeinheit auf einem gemeinsamen Träger angeordnet sein. Die Antenneneinheit erlaubt dabei das Auslesen der gespeicherten Information der Chipeinheit. Dabei sind vorzugsweise Funkantennen gemeint, wie sie typischerweise bei RFID (Radio Frequency Identification)-Transpondern oder NFC (Near Field Communication) Tags eingesetzt werden. Insbesondere durch induktive Kopplung auslesbare Elemente können als Antenneneinheit im vorliegenden Sinne angesehen werden. Des Weiteren kann es zweckmäßig sein, wenn die Antenneneinheit ein Trägerelement zur Aufnahme der Leiterbahn umfasst

Der Transmitter und/oder die Antenne können als Teil einer Sende- und Empfangseinheit dazu geeignet sein, Daten, Signale und/oder Energie berührungslos bzw. drahtlos unidirektional, vorzugsweise bidirektional zu übertragen.

Wenn nachfolgend Eigenschaften des Sensors beschrieben werden oder der Begriff Sensor verwendet wird, so gilt diese Beschreibung auch für ein anderes Funktionselement, welches im Bereich der Deckschicht, insbesondere der dem Produkt zugewandten oder abgewandten Deckschicht angeordnet bzw. verbunden ist

Mit Vorteil ist der Sensor zur Erfassung wenigstens einer Eigenschaft eines in der Verpackung abgepackten, insbesondere zumindest teilweise schüttfähigen, pastösen und/oder flüssigen Produktes eingerichtet. Der Sensor kann dabei unmittelbar zumindest in Teilen auf der äußeren Seite der Deckschicht angeordnet sein oder die Deckschicht in Richtung des Produktes durchbrechen. Somit kann der Sensor zur direkten Erfassung der wenigstens einen Eigenschaft des Produktes geeignet sein. Der Sensor kann auch zumindest in Teilen innerhalb der Deckschicht und insbesondere zwischen der Deckschicht und der Trägerschicht vorgesehen sein. Der Sensor kann dann zur indirekten Erfassung wenigstens einer Eigenschaft des Produktes geeignet sein.

Der Sensor kann zur Messung des pH-Werts, der Temperatur, des Sauerstoffgehalts, des Anteils eines oder mehrerer Vitamins/Spurenelements, der elektrischen Leitfähigkeit, von Stoffwechselprodukten oder dergleichen eingerichtet sein. Es kann von Vorteil sein, wenn der Sensor zur Ermittlung wenigstens eines Absolutwertes gebildet ist.

Es ist von Vorteil, wenn zur Ermittlung der augenblicklich vorliegenden Qualität des Lebensmittels zwei Messwerte herangezogen werden. Dabei kann es sich um denselben Parameter handeln, wobei der Sensor den Messwert, bspw. einen pH-Wert, erstmalig z.B. beim Inkontakttreten mit dem Produkt misst und danach eine Veränderung des Messwertes, ohne den tatsächlichen quantitativen Wert zu kennen. Der Sensor ist daher bevorzugt ein Sensor, der eine qualitative Veränderung eines Messwertes erfasst Daher kann es fallweise von Vorteil sein, wenn der Sensor zur Ermittlung wenigstens eines Relativwertes des Messwertes relativ zu einem initialen Messwert gebildet ist

Auch kann mehr als ein Sensor zur Ermittlung unterschiedlicher Parameter verwendet werden. Da sich beispielsweise die Temperatur des untersuchten Produkts auf die Wertermittlung des Sensors heute noch häufig auswirkt, ist es für die Korrektheit einer daraus abgeleiteten Zustandsbestimmung der Qualität wichtig, dass die Wertermittlung anhand der vorliegenden Temperatur beurteilt wird. Daher wird vorgeschlagen, dass ein Messwert eines ersten Sensors abhängig von einem Messwert eines zweiten Sensors, insbesondere einem Temperaturwert eines Temperatursensors, gewichtet und/oder normiert wird.

Mit Vorteil umfasst der Sensor einen ersten Bereich und einen zweiten Bereich, wobei der erste Bereich offen gegenüber seiner Umgebung, insbesondere die Deckschicht in Richtung des Produktes durchbrechend gebildet ist und der zweite Bereich wenigstens teilweise isoliert gegenüber seiner Umgebung, insbesondere durch die Deckschicht gegenüber dem Produkt abgedeckt gebildet ist

Mit Vorteil bildet wenigstens ein Teil der Oberfläche der dem Produkt zugewiesenen Deckschicht und/oder des Sensors einen für zumindest einen zu detektierenden Gegenstand bevorzugten Aufenthaltsbereich.

Es sind zudem Ausführungen von teilweise bzw. vollständig druckbaren Sensoren denkbar, welche auf dem Prinzip eines Ionensensitive Feldeffekttransistors (ISFET) bzw. Electrolyte-Insulator-Semiconductor (EIS) beruhen. Hierbei werden die Standardmaterialien der Siliziumtechnologie ganz oder teilweise durch funktionale Tinten auf Basis von Polymeren ersetzt, welche die gleichen bzw. ähnliche elektrische bzw. elektrochemische Eigenschaften aufweisen wie die herkömmlichen Materialien. Die Strukturen werden dabei mit unterschiedlichen Druckverfahren, z.B. Siebdruck oder Ink-Jet-Druck auf einem Substrat, insbesondere der Deckschicht erzeugt. Das Substrat kann dabei neben seiner Funktion als Träger der Sensorstruktur selbst als funktionaler Teil der Sensorstruktur dienen, z.B. als Isolator, Halbleiter oder ionensensitive Schicht Als Substrat können dabei einschichtige, mehrschichtige, steife oder flexible Träger in Form von z.B. Folien, Platten (Plättchen), Bögen, Streifen o.Ä. dienen. Insbesondere die Kombination aus teilweise bzw. vollständig gedruckten Sensorstrukturen aus funktionalen Tinten auf flexiblen Foliensubstraten als Träger ermöglicht eine kostengünstige Fertigung, sogar bei kleineren Stückzahlen.

Eine kostengünstige Bereitstellung eines Sensors ist im Zusammenhang mit der vorliegenden Erfindung deshalb so besonders wichtig, weil der Packstoff, respektive die Bahnware zur Herstellung einer Verbundverpackung dient. Eine Verbundverpackung soll im Sinne der vorliegenden Erfindung insbesondere zum Schutz eines frisch oder bevorzugt aseptisch abgefüllten Lebensmittels dienen und wird dem zufolge nach Verzehr des Lebensmittels i.d.R. entsorgt. Sind sogar für kleine Stückzahlen preiswerte Sensoren verfügbar, können auch entsprechende Ausführungsformen von Packstoff für seltenere Packungsformen und/oder für Packungen für seltenere Lebensmittel preiswert erzeugt werden.

Hinsichtlich der Sensoren, die mit einem potentiometrischen Messverfahren arbeiten stellt jedoch das zwingende Erfordernis einer Referenzelektrode einen generellen Nachteil dar. Eine Referenzelektrode wird hierbei benötigt, um ein konstantes Potential bereitzustellen, gegen welche das Elektrodenpotential der Messelektrode abgegriffen werden kann. Die häufigste Form der Referenzelektrode ist eine Silber-Silberchlorid- (Ag/AgCl) Elektrode.

Referenzelektroden haben den Nachteil, dass je kleiner sie gebaut, desto instabiler die von ihnen gelieferten Gleichgewichtspotentiale sind. Zudem sind die meisten Materialpaarungen, z.B. Ag/AgCl, unerwünscht, wenn sie in Kontakt mit Lebensmitteln treten, insbesondere dann, wenn Chloride in das Lebensmittel eingebracht werden könnten (z.B. weil das Lebensmittel besonders aufnahmefähig für Chloride ist oder bestimmte andere Parameter erfüllt sind).

Deshalb ist mit Vorzug dafür gesorgt, dass der Sensor mit einer sogenannten Pseudo-Referenzelektrode als Ersatz für eine Referenzelektrode ausgestattet ist Hierbei handelt es sich um einfache Metalldrähte bzw. Metalloberflächen, an denen sich ebenfalls ein konstantes, jedoch unbekanntes Potential in einer Elektrolytlösung einstellt Die Messung der Potentialdifferenz wird dadurch ungenauer im Vergleich zu einer z.B. Ag/AgCl-Referenzelektrode. Diese Variante wird insbesondere bei Anwendungen bevorzugt, bei denen ein Übergang von z.B. Chloriden in das Messmedium zu vermeiden ist, insbesondere bei Bedarfsgegenständen.

Ein Messaufbau der ohne Referenzelektrode auskommt beruht auf dem Prinzip des Redoxcyclings und ist deshalb besonders bevorzugt Hierbei wird durch wechselseitiges beaufschlagen eines Potentials an zwei Elektroden in der Analytlösung, beispielsweise dem zu analysierenden Produktes, insbesondere Lebensmittel beziehungsweise einer Komponente oder eines Parameters des zu analysierenden Lebensmittels, ein messbarer Strom erzeugt, der durch Redoxreaktionen des Analyten an den Elektroden entsteht. Der messbare Strom steht dabei in mittelbarem bzw. unmittelbarem Zusammenhang mit der Analytkonzentration. In einer besonders einfachen Ausführungsform besteht ein dementsprechender Sensor aus genau zwei Elektroden. Es sind aber auch Mehrelektrodenanordnungen, auch unter Einbezug einer Referenzelektrode denkbar. Die Elektroden können dabei als einfache Metalldrähte oder ganz bzw. teilweise strukturierte, flächig ausgebrachte Elektrodenstrukturen ausgeführt sein. Zur Herstellung der Elektrodenstrukturen können beispielsweise die analog zu den ionensensitiven Feldeffekttransistoren (ISFET) und den EIS- (Electrolyte Insulator Semiconductor) Sensoren beschriebenen Herstellungsverfahren und Trägermaterialien (Substrate) verwendet werden. Auch hier lassen sich insbesondere durch die Verwendung von funktionalen Tinten in Kombination mit verschiedenen Drucktechniken kostengünstige Sensorstrukturen auf Foliensubstraten, insbesondere der Deckschicht realisieren.

Mit Vorteil ist der Sensor als Temperatursensor ausgebildet Dabei kann es sich fallweise mit Vorteil um einen aktiven Temperatursensor handeln. Aktive Temperatursensoren erzeugen aufgrund ihres Messprinzips ein elektrisches Signal. Dies hat den Vorteil, dass dabei keine elektrische Hilfsenergie benötigt wird. Ein Beispiel für einen solchen aktiven Temperatursensor ist das Thermoelement Insbesondere in diesem Fall kann auch bereits ein elektrisch gegenüber der Barriereschicht isolierter Leiter ausreichend sein.

In anderen Fällen kann es bevorzugt sein, dass der Temperatursensor als passiver Temperatursensor ausgebildet ist. Bei passiven Temperatursensoren wird im Gegensatz zu aktiven Sensoren eine Hilfsenergie benötigt, um das Signal auslesen zu können. Beispiel für einen passiven Temperatursensor ist das Widerstandsthermometer. Es handelt sich hierbei um elektrisches Bauelement, welches die Temperaturabhängigkeit des elektrischen Widerstandes eines elektrischen Leiters zur Messung der Temperatur ausnutzt. Als Widerstandsmaterial eignen sich vorzugsweise reine Metalle. Sie zeigen stärkere Widerstandsänderungen als Legierungen. Ferner haben sie einen nahezu linearen Zusammenhang des Widerstandes mit der Temperatur. Häufig wird Platin verwendet

Es können jedoch auch Messwiderstände aus Keramik (gesinterte Metalloxide) oder Halbleitern verwendet werden. Damit lassen sich sehr viel höhere Temperaturkoeffizienten als mit Metallen und damit auch viel höhere Empfindlichkeiten erzielen, teilweise jedoch zu Lasten der Genauigkeit

Sowohl Thermoelemente als auch verschiedene Ausführungen von Widerstandsthermometern lassen sich heute auf drucktechnischem Wege kostengünstig herstellen.

Mit Vorteil handelt es sich bei wenigstens einem des wenigstens einen Sensors um einen Leitfähigkeitssensor.

Mit einem Leitfähigkeitssensor lässt sich die elektrische Leitfähigkeit insbesondere von Flüssigkeiten bestimmen. Die Leitfähigkeit ergibt sich dabei als Summenparameter aller in der Flüssigkeit gelöster, dissoziierter Stoffe (Ionen). Dies ist von besonderem Vorteil, wenn aus der Bahnware (Packstoff) eine Verpackung erzeugt ist, die zur Bewahrung eines wenigstens eine Flüssigkeitskomponente aufweisenden Produkts, vorzugsweise eines Lebensmittels, dient. Hier macht die Weiterbildung sich zu Nutze, dass der elektrische Widerstand bei den meisten flüssigen oder wenigstens eine Flüssigkeitskomponente aufweisenden Lebensmitteln mit Übergang aus einem bedenkenlos genießbarem Zustand und einem verdorbenen Zustand ändert

Der Leitfähigkeitssensor besteht beispielsweise aus zwei zueinander parallel oder koaxial angeordneten Elektroden. Die Elektroden bestehen aus Edelstahl, Graphit oder, eher selten, aus Reinmetallen wie Platin oder Titan. Die Elektroden besitzen eine definierte Fläche und stehen in einem definierten Abstand zueinander. Die Flüssigkeit zwischen den Elektroden verhält sich dabei wie ein ohmscher Widerstand, der z.B. nach einem konduktiven Messverfahren ausgelesen werden kann. Es gibt auch Messschaltungen nach dem induktiven Verfahren, die den Vorteil bieten, dass sie potentialfrei von der Flüssigkeit betrieben werden können.

Einfache Elektrodenstrukturen zur Leitfähigkeitsmessung lassen sich auf drucktechnischem Wege kostengünstig herstellen.

Mit Vorteil handelt es sich bei wenigstens einem des wenigstens einen Sensors um einen Sauerstoffsensor.

Durch das Vorsehen eines Sensors wird es möglich, eine oder mehrere Eigenschaften beispielsweise eines, mit dem Bahnmaterial, insbesondere mit ihrer dem Produkt zugewiesenen Deckschicht in Kontakt tretenden Produktes zu ermitteln. Um verschiedene Eigenschaften ermitteln zu können, ist es selbstverständlich denkbar, dass der das Funktionselement mehrere, insbesondere unterschiedliche Sensoren umfasst und/oder, dass das Funktionselement noch weitere Teile umfasst, die dann beispielsweise ebenfalls einen Sensor umfassen.

Mit Vorteil handelt es sich bei wenigstens einem des wenigstens einen Sensors um einen pH-Sensor.

Der pH-Wert ist ein Maß für den sauren oder basischen Charakter einer wässrigen Lösung. Er wird anhand der Wasserstoffionenaktivität der wässrigen Lösung ermittelt Es gibt verschiedene Methoden den pH-Wert zu ermitteln. Eine Möglichkeit liegt in der Anwendung einer potentiometrischen Messkette (Potentiometrie). Hierbei wird das Potential, welches sich in direkter Abhängigkeit zur H+-Ionen-Konzentration an einer ionensensitiven Elektrode bildet, gemessen. Die Messung erfolgt dabei als Potentialdifferenzmessung gegenüber einer Bezugselektrode, auch Referenzelektrode genannt, welche ein konstantes Potential bereitstellt. Heute ist die pH-Glaselektrode als eine Ausführungsform einer pH-Elektrode besonders gut am Markt verfügbar. Sie wird meist als Einstabmesskette mit integrierter Referenzelektrode gebaut.

Neben Glaselektroden existieren weitere Ausführungsformen von potentiometrischen pH-Sensoren die ebenfalls mit Vorzug im Zusammenhang mit der vorliegenden Erfindung Anwendung finden können. Dabei handelt es sich beispielsweise um den ionensensitive Feldeffekttransistor (ISFET) und den EIS- (Electrolyte Insulator Semiconductor) Sensor. Beim ISFET handelt es sich um eine Sonderform des Feldeffekttransistors, bei dem der Gatekontakt durch ein ionen- bzw. pH-sensitives Material ersetzt wird (z.B. SiO₂, Al₂O₃ oder Ta₂O₅). Der EIS Sensor gleicht im Prinzip einer Metall-Isolator-Halbleiter-Struktur, wobei der Metallkontakt durch den Messelektrolyten und die Referenzelektrode und der Isolator durch eine ionensensitive Schicht (z.B. SiO₂, Al₂O₃ oder Ta₂O₅) ersetzt werden. Ein Vorteil dieser Ausführungsformen liegt u.a. in der Möglichkeit zur Miniaturisierung.

ISFET bzw. EIS Strukturen können unter Anwendung der Siliziumtechnologie hergestellt werden. Bei hohen Produktionsstückzahlen können somit Kostenvorteile bei der Herstellung der Sensoren erzielt werden.

Das erste und/oder das elektrische Element kann mit einem Speicher ausgestattet sein. Als Speicher ist dabei ein Datenspeicher zu verstehen. Dabei handelt es sich um ein Speichermedium welches der Speicherung von elektronischen Daten dient Der Datenspeicher kann dabei wahlweise als flüchtiger Speicher oder als nicht-flüchtiger Speicher bzw. als eine Kombination aus beiden genannten Speichertypen ausgelegt sein. Nicht-flüchtige Speicher werden noch in permanente bzw. semi-permanente Speicher unterteilt.

Als flüchtige Speicher sind hierbei Speicher zu verstehen, deren Informationen verloren gehen, wenn sie nicht aufgefrischt werden oder wenn der Strom abgeschaltet wird. Als nicht-flüchtiger Speicher sind Speicher zu verstehen, bei denen die gespeicherte Information längere Zeit (mindestens Monate) ohne Anliegen einer Betriebsspannung erhalten bleibt. Bei permanenten Speichern bleibt die einmal gespeicherte oder festverdrahtete Information vorhanden und kann nicht mehr verändert werden. In semi-permanenten Speichern lassen sich Informationen permanent speichern, jedoch können die Informationen auch noch verändert werden.

Der jeweilige Speichertyp soll nach dem Anwendungsfall gewählt sein, dem die aus der Bahnware zu erzeugende Verpackung einmal dienen soll. Dabei ergeben sich je nach Anwendungsfall bestimmte Vorteile

Der Datenspeicher kann ist Teil der Antenne oder des Transmitters sein. Der Datenspeicher kann Teil eines sogenannten "Tags" sein, welches mittels Funktechnik auslesen lässt, insbesondere als RFID Tag. Der Datenspeicher kann bereits während des Produktionsprozesses des Packstoffs ganz oder teilweise beschrieben werden. Der Datenspeicher kann teilweise oder ganz beschrieben sein, bevor er in das Laminat integriert wird. Der Datenspeicher kann mit einer eindeutigen ID beschrieben sein. Der Datenspeicher kann ganz oder teilweise mit Produktionsdaten beschrieben sein. Der Datenspeicher kann ganz oder teilweise an der Füllmaschine beschrieben werden. Der Datenspeicher kann ganz oder teilweise an einem oder mehreren Punkten der Wertschöpfungskette beschrieben werden. Der Datenspeicher kann ganz oder teilweise zu einem oder mehreren Zeitpunkten beschrieben werden. Die auf dem Datenspeicher gespeicherten Informationen können ganz oder teilweise ersetzt werden.

Mit besonderem Vorteil umfasst das elektrische Element zumindest in Teilen eine Sende- und Empfangseinheit mit einer Antenne und/ oder einem Speicher und/oder einen Sensor.

Gemäß einem Ausführungsbeispiel wird vorgeschlagen, dass die Barriereschicht zumindest einen Teil zumindest eines der elektrischen Leiter bildet Insbesondere kann die Barriereschicht einen der elektrischen Leiter selbst bilden. Die Barriereschicht ist vorzugsweise metallisch, um eine Gasbarriere zu bilden. Dieser Umstand kann genutzt werden, um eine elektrische Leitung von der einen Seite der Barriereschicht zu der anderen Seite der Barriereschicht zu ermöglichen. Wie bereits erläutert, sind zwei elektrische Leiter bevorzugt, die elektrisch voneinander isoliert sind und von der einen Seite der Barriereschicht zu der anderen Seite der Barriereschicht reichen. Einer dieser Leiter kann die Barriereschicht selbst sein. In diesem Fall kann ein erster Leiter isoliert durch die Barriereschicht geführt werden. Die Barriereschicht selbst kann den zweiten Leiter bilden. Das Funktionselement kann einerseits mit einem der Leiter verbunden sein oder diesen bilden und andererseits mit der Barriereschicht verbunden sein. Das elektrische Element kann mit der Barriereschicht verbunden sein, so dass ein elektrischer Pfad über die Barriereschicht zwischen dem Funktionselement und dem elektrischen Element gebildet ist. Ein zweiter elektrischer Pfad zwischen dem Funktionselement und dem elektrischen Element kann durch den gegenüber der Barriereschicht isolierten Leiter gebildet sein.

Es versteht sich, dass das Funktionselement mit dem elektrischen Element über die beiden Leiter nur dann verbunden ist, wenn die elektrischen Leiter jeweils mit Kontakten der Elemente verbunden sind. Somit ist bevorzugt, wenn jeweils ein Kontakt eines der Elemente mit jeweils zumindest einem der Leiter kontaktiert ist Die Kontakte der Elemente können zumindest in Teilen die Leiter selbst bilden.

Gemäß einem Ausführungsbeispiel wird vorgeschlagen, dass die Barriereschicht eine Ausnehmung aufweist und dass zumindest einer der elektrischen Leiter durch die Ausnehmung geführt ist. Bei der Herstellung des Packstoffs wird dieser in Transportrichtung mit den jeweiligen Schichten laminiert. Dabei ist es möglich, dass im Verlaufe des Transports des Packstoffes beispielsweise eine Ausnehmung in einen definierten Bereich der Barriereschicht eingebracht, insbesondere gestanzt wird. Auch kann die Ausnehmung mittels eines Lasers aus der Barriereschicht herausgeschnitten werden. Bevorzugt ist der Bereich, in dem die Ausnehmung in die Barriereschicht eingebracht ist, derart, dass dieser in der Packung im Bereich einer Nahtstelle, insbesondere im Bereich einer Überlappung zweier Kanten des Zuschnitts liegt Insbesondere liegt die Ausnehmung im Bereich einer Längskante oder einer Querkante des Zuschnitts, aus dem die Packung erstellt wird. Die Ausnehmung ist vorzugsweise kreisförmig, kann jedoch auch eine andere Form haben. Die Ausnehmung hat vorzugsweise einen Durchmesser von weniger als 5 mm, vorzugsweise weniger als 1 mm. Da die Leiter nur eine geringe Stromtragfähigkeit aufweisen müssen, können diese Durchmesser von weniger als 5 mm, vorzugsweise weniger als 1 mm aufweisen. Somit kann die Ausnehmung so gestaltet sein, dass sich zwischen der Barriereschicht und dem in der Ausnehmung geführten Leiter ein Ringraum bildet. Dieser Ringraum bildet die Isolation des Leiters gegenüber der Barriereschicht Durch die Ausnehmung kann sichergestellt werden, dass ein Leiter isoliert gegenüber der Barriereschicht durch die Barriereschicht geführt wird.

Gemäß einem Ausführungsbeispiel wird vorgeschlagen, dass das Funktionselement mit einer Schicht auf der ersten Seite der Barriereschicht verbunden ist. Auch wird vorgeschlagen, dass das elektrische Element mit einer Schicht auf der zweiten Seite der Barriereschicht verbunden ist.

Es ist möglich, dass Sensoren und/oder Antennen gedruckt werden, wie oben bereits beschrieben. Insbesondere ist es möglich, einen Sensor oder ein anderes Funktionselement in oder auf der Deckschicht, welche dem Produkt zugewandt ist, zu verbinden. Insbesondere ist es möglich, eine Antenne oder ein anderes elektrisches Element in oder auf der Deckschicht, welche dem Produkt abgewandt ist, zu verbinden. Auch sind elektrische Elemente bekannt, die bereits als folienartiges Bauteil erhältlich sind. So bereits vorlaminierte elektrische Elemente können auch Sensoren oder Antennen enthalten. Dieses folienartige Element kann mit auf einer Seite der Barriereschicht verbunden werden. Es ist möglich, dass dies unmittelbar auf der Barriereschicht oder auf der Deckschicht oder einer dazwischen liegenden Schicht erfolgt Mit einer Schicht verbunden kann in dieser Offenbarung auf, in oder an einer Schicht angeordnet bedeuten. Anordnen kann durch Auf-/Einlaminieren, Drucken, Kleben, Nieten, Stecken oder dergleichen erfolgen.

Bei der Herstellung der Bahnware kann es möglich sein, laminierte elektrische Elemente, die beispielsweise als Folienelemente vorliegen, von einer Rolle abzuwickeln und im Herstellungsprozess in einem kontinuierlichen Aufbringungsverfahren auf den im Herstellungsprozess sich bewegenden Packstoff auf zu laminieren. Eine Vielzahl Funktionselementen und/oder elektrischer Elemente können als Bahnware von einer Rolle abgewickelt werden. Die auf der Rolle bzw. der Bahnware angeordneten Elemente können einen definierten Abstand zueinander haben, so dass die Elemente im Zuschnitt jeweils an einer gleichen Position angeordnet ist.

Es ist auch erkannt worden, dass zur Bereitstellung der gewünschten Informationen für den Benutzer wenigstens ein Funktionselement in das Packungslaminat integriert werden kann, und zwar zwischen die Trägerschicht und eine Deckschicht und/oder wenigstens teilweise in die Trägerschicht bzw. Deckschicht. Dies vereinfacht die Herstellung des Packungslaminats und die Anbringung des Elements. Hier kann sich nämlich die strukturgebende und verhältnismäßig biegesteife Trägerschicht genutzt werden. Die Trägerschicht bildet so ein geeignetes Substrat zur Anbringung und Aufnahme des wenigstens einen Elements. Alternativ oder zusätzlich stellt die Trägerschicht eine Schutzwirkung im Hinblick auf das Element bereit, so dass dieses auf Dauer beschädigungsfrei im Packungslaminat aufgenommen werden kann. So kann beispielsweise das Packungslaminat nach seiner Herstellung problemlos zu einer Rolle aufgerollt werden, um sodann an einen anderen Ort verbracht zu werden. Die Trägerschicht schützt des wenigstens eine elektronische Funktionselement vor Beschädigung, insbesondere durch Knicken oder übermäßiges Umbiegen. Das Element kann aber auch bei der Bildung der Packung und in der Packung selbst durch die Trägerschicht geschützt werden. Bei der Integration des Elements wenigstens teilweise in die Deckschicht kann die Biegesteifigkeit der Trägerschicht ebenfalls genutzt werden. Zudem kann die Schichtdicke des Packungslaminats gering gehalten werden, ohne das Element wenigstens teilweise in die Trägerschicht aufnehmen zu müssen. Hinzu kommt, dass so bedarfsweise ein Kontakt des Elementes zur Außenseite des Packungslaminats bzw. zur Innenseite der späteren Packung bereitgestellt werden kann.

Gemäß einem Ausführungsbeispiel wird vorgeschlagen, dass die Barriereschicht zumindest entlang einer Richtung durchgehend in zwei voneinander getrennte Bereiche aufgetrennt ist So ist es möglich, mit einem Messer oder einem Laser entlang der Bewegungsrichtung der Bahnware eine durchgehende Auftrennung der Barriereschicht vorzunehmen. Im Zuschnitt kann diese Auftrennung entweder im Bereich einer Längsnaht oder im Bereich einer Quernaht vorliegen. Dies ist abhängig davon, ob die Zuschnitte, nachdem sie aus der Bahnware hergestellt wurden, um 90° zur Längsrichtung gedreht werden oder nicht Im Regelfall erfolgt eine Drehung um 90°. In diesem Fall wäre eine Auftrennung in Längsrichtung, d.h. in Bewegungsrichtung der Bahnware im Zuschnitt einer Auftrennung nicht entlang der Längsnaht, sondern entlang der Quernaht. Es ist jedoch auch möglich, dass die Auftrennung der Barriereschicht in zwei voneinander getrennte Bereiche erst nach Herstellung des Zuschnitts erfolgt. Auch die Zuschnitte können kontinuierlich unter einem Messer oder einem Laser bewegt werden. Dadurch können auch die Zuschnitte dazu genutzt werden, die Barriereschicht entlang einer Richtung durchgehend in zwei voneinander getrennte Bereiche aufzutrennen. Die Auftrennung kann vorzugsweise parallel zu einer Kante, insbesondere zur Längskante oder zur Querkante des Zuschnitts verlaufen.

Ist eine solche Auftrennung der Barriereschicht erfolgt, kann diese zumindest Teile jeweils eines der beiden elektrischen Leiter bilden. Ein erster Bereich kann Teil eines ersten elektrischen Leiters bilden und ein zweiter Bereich kann zumindest Teil eines zweiten elektrischen Leiters bilden. Somit kann beispielweise das Funktionselement mit seinen elektrischen Anschlüssen unmittelbar jeweils auf einem der Bereiche der Barriereschicht kontaktiert werden und das elektrische Element kann ebenfalls mit jeweils einem der Anschlüsse jeweils auf einem der Bereiche kontaktiert werden. Dann wird die elektrische Leitung durch die Barriereschicht über die Barriereschicht selbst ermöglicht, wobei die Barriereschicht zwei elektrische Potentiale tragen kann.

Der Packstoff ist aus einem Laminat aus mehreren Schichten umfassend zumindest eine Trägerschicht, eine Barriereschicht und eine Deckschicht gebildet. Elektrische Leiter können isoliert auf einer dieser Schichten gegenüber der Barriereschicht geführt werden. So ist es möglich, einen elektrischen Leiter auf einer Deckschicht zu führen. Auch ist es möglich, einen elektrischen Leiter auf einer Trägerschicht zu führen. Der elektrische Leiter ist dabei bevorzugt gegenüber der Barriereschicht isoliert.

Darüber hinaus kann es sinnvoll sein, dass ein Teil des Leiters durch ein Durchdringungselement gebildet ist Das Durchdringungselement kann dabei die Leiterbahn zumindest teilweise durchdringen. Auch kann das Durchdringungselement zumindest die Barriereschicht durchdringen. Das Durchdringungselement kann stiftförmig oder nadelförmig sein. Insbesondere kann das Durchdringungselement einen tellerförmigen Kopf und einen davon abstehenden Stift aufweisen. Mit dem tellerförmigen Kopf kann das Durchdringungselement an der Leiterbahn anliegen und mit dem Stift kann das Durchdringungselement die Barriereschicht berühren oder durchdringen. Insbesondere kann das Durchdringungselement in dem Bereich angeordnet sein, in dem die Barriereschicht seine Ausnehmung, wie oben erläutert, aufweist.

Auch ist es möglich, dass das Durchdringungselement in Kontakt mit Leiterbahnen auf beiden Seiten der Barriereschicht steht So ist es möglich, dass eine erste Leiterbahn mit dem Funktionselement verbunden ist oder Teil davon ist und eine zweite Leiterbahn mit dem elektrischen Element verbunden ist oder Teil davon ist Das Durchdringungselement kann dann durch beide Leiterbahnen und die Barriereschicht durchdringen und somit die Leiterbahnen miteinander elektrisch verbinden. Das Durchdringungselement ist dabei bevorzugt metallisch. Insbesondere ist das Durchdringungselement aus dem selben Metall gebildet, wie die Leiterbahnen. Ist die Barriereschicht Teil eines Leiters, so kann es ausreichend sein, wenn das Durchdringungselement die Barriereschicht berührt In diesem Fall kann das Durchdringungselement einen elektrischen Kontakt zwischen der Leiterbahn und der Barriereschicht herstellen. Vorzugsweise ist der tellerförmige Kopf auf der Seite der Barriereschicht, der im Inneren der Packung ist Somit ist das Durchdringungselement von innen nach außen einer Packung durch die Leiterbahn und zumindest in Teilen die Barriereschicht durchgesteckt

Das Durchdringungselement kann ebenfalls von einer Rolle abgerollt werden und während des Herstellungsverfahrens des Packstoffes in einem kontinuierlichen Prozess an den geeigneten Positionen der Bahnware angebracht werden. So ist es möglich, dass zunächst die Leiterbahnen, die Folienleiter sein können oder aufgedruckt sein können, auf beispielsweise die innere Deckschicht und die Trägerschicht aufgebracht werden. Dabei ist es bevorzugt, wenn die Leiterbahnen auf einander gegenüberliegenden Seiten übereinander liegend, zueinander fluchtend auf dem Packstoff aufgebracht sind. So kann das Durchdringungselement senkrecht zur Oberfläche des Packstoffes durch zumindest die Barriereschicht zumindest in Teilen durchgesteckt werden.

Gemäß einem Ausführungsbeispiel wird vorgeschlagen, dass auf einer ersten Schicht auf der ersten Seite der Barriereschicht zumindest eine elektrisch leitende Leiterbahn aufgebracht ist und dass ein Durchdringungselement die Leiterbahn zumindest teilweise durchdringt und/oder die Schichten auf der ersten Seite der Barriereschicht zumindest teilweise durchdringt.

Gemäß einem Ausführungsbeispiel wird vorgeschlagen, dass auf einer Schicht auf der ersten Seite der Barriereschicht und einer Schicht auf der zweiten Seite der Barriereschicht jeweils zumindest einer der elektrischen Leiter bzw. Leiterbahnen aufgebracht ist, wobei die jeweiligen elektrischen Leiter bzw. Leiterbahnen einander überlappen. Das bedeutet, dass in der Normalprojektion der Schichten parallel zur Flächennormale der Schichten die jeweiligen Leiter bzw. Leiterbahnen übereinander liegend. Dies vereinfacht die Kontaktierung der Leiter bzw. Leiterbahnen. So können mit Hilfe eines Durchdringungselementes, welches parallel zur Flächennormale des Packstoffs durch zumindest die Barriereschicht oder eine andere Schicht des Packstoffes durchgesteckt ist, die beiden Leiter bzw. Leiterbahnen auf den jeweiligen Seiten der Barriereschicht miteinander elektrisch verbunden werden.

Auch ist es möglich, dass nachdem der Zuschnitt aus dem Packstoff erstellt wurde, zwei gegenüberliegende Kanten des Zuschnittes überlappend übereinander gelegt werden. In diesem Fall, in dem die Kanten überlappend übereinander gelegt werden, kann dann durch diese zuvor beschriebene Anordnung der Leiter bzw. Leiterbahnen sichergestellt werden, dass ein erster Leiter bzw. Leiterbahn, welche auf der ersten Seite der Barriereschicht angeordnet ist, im Bereich der Überlappung mit dem zweiten Leiter bzw. der zweiten Leiterbahn, die auf der zweiten Seite der Barriereschicht angeordnet ist, unmittelbar in Kontakt steht Hierbei verlaufen die überlappenden Kanten parallel zueinander. Somit ist ohne eine Durchdringung der Barriereschicht eine Kontaktierung von innen nach außen in der Packung möglich. Eine Versiegelung der Kante kann durch einen Versiegelungsstreifen erfolgen, der auch über die Leiterbahn bzw. den Leiter gelegt ist, der auf der Innenseite der Packung liegt Auch kann die Versiegelung durch Heißsiegeln der aufeinander liegenden Deckschichten erfolgen. Auch können die Leiter mit einer Schicht isoliert sein, welche siegelfähig ist und mit den anliegenden Deckschichten versiegelt wird.

Eine erste Seite der Barriereschicht kann in der Packung eine Innenseite sein. Auf einer solchen ersten Seite kann eine Schicht die Deckschicht sein, die dort unmittelbar auf der Barriereschicht aufgebracht ist. Es ist möglich, dass das Funktionselement zumindest teilweise und/oder einer der elektrischen Leiter bzw. Leiterbahnen zwischen der Barriereschicht und der Deckschicht aufgebracht sind. In diesem Fall kann der elektrische Leiter beispielsweise als Folienleiter gebildet sein und somit gegenüber der Barriereschicht selbstständig isoliert sein. Auch das Funktionselement, beispielsweise der Sensor, kann als Folienelement gebildet sein, wobei die Elektronik in einer Folie einlaminiert ist. Diese Folie kann auf die Barriereschicht aufgelegt werden und es besteht durch die Folien Funktionselements eine elektrische Isolation des Funktionselements gegenüber der Barriereschicht Die Folie kann siegelfähig mit zumindest einer der Deckschichten sein, insbesondere aus dem gleichen Kunststoff gebildet sein.

Auch ist es möglich, dass das Funktionselement und/oder einer der elektrischen Leiter auf der der Barriereschicht abgewandten Seite der Deckschicht aufgebracht sind. Dies kann insbesondere dann sinnvoll sein, wenn das Funktionselement und/oder der elektrische Leiter bzw. die Leiterbahn nicht selbstständig isoliert sind. Dann kann die Deckschicht eine Isolation des elektrischen Leiters, der Leiterbahn und/oder des Funktionselements gegenüber der Barriereschicht bilden. Dies ist insbesondere für aufgedruckte elektronische Elemente, Leiter oder Leiterbahnen eine sinnvolle Anordnung. Auch kann eine Haftvermittlerschicht als Isolationsschicht dienen.

Auch für den oben genannten Fall des Durchkontaktierens über einen Überlappungsbereich, in dem im Überlappungsbereich zwei Kanten des Packstoffs sich überlappen, insbesondere eine erste Seite des Packstoffs auf einer zweiten Seite des Packstoffs aufliegt, insbesondere eine Innenseite auf einer Außenseite, kann es sinnvoll sein, den elektrischen Leiter bzw. die Leiterbahn auf der der Barriereschicht abgewandten Seite der Deckschicht aufzubringen. Die Leiterbahn bzw. der Leiter kann dann unmittelbar auf der Oberfläche angeordnet sein, die dem Produkt zugewandt ist. Im befüllten Zustand kann dann der Leiter bzw. die Leiterbahn in unmittelbaren Kontakt mit dem abgefüllten Produkt sein.

Eine zweite Seite der Barriereschicht kann in der Packung einer Außenseite zugewandt sein. Auf dieser zweiten Seite der Barriereschicht ist zumindest eine Trägerschicht aufgebracht. Es versteht sich, dass die Reihenfolge, in der die Schichten aufeinander aufgebracht sind, insbesondere auch zunächst die Trägerschicht umfassen kann, auf die die Barriereschicht dann aufgebracht ist, was mit der obigen Formulierung ebenso gemeint ist Das elektrische Element, eine Leiterbahn und/oder einer der elektrischen Leiter kann zwischen der Barriereschicht und der Trägerschicht oder auf einer Schicht, die auf der der Barriereschicht abgewandten Seite der Trägerschicht angeordnet ist, aufgebracht sein. Eine Schicht, die auf der der Barriereschicht abgewandten Seite der Trägerschicht angeordnet ist, kann beispielsweise die äußere Seite der Trägerschicht, eine auf der Trägerschicht aufgebrachte Deckschicht, eine Farbschicht oder dergleichen sein. Insbesondere wenn eine Leiterbahn oder ein elektrischer Leiter auf einer solchen äußeren Schicht aufgebracht ist, kann im Fall des Überlappens des Zuschnitts bzw. zweier Kanten des Zuschnitts eine Durchkontaktierung von innen nach außen erfolgen, in dem die Leiter oder Leiterbahnen aufeinander aufliegen.

Die erste Seite der Barriereschicht ist der Innenschicht einer Packung und die zweite Seite der Barriereschicht ist einer Außenschicht der Packung zugewandt ist

Der Packstoff wird zunächst als Bahnware mit relativ breiten Bahnen in einem Endlosprozess hergestellt Dabei werden auf die Trägerschicht in aufeinanderfolgenden Laminierprozessen die Barriereschicht und die innere und äußere Deckschicht aufgetragen. Nachdem alle Schichten des Packstoffes auflaminiert wurden, wird die Bahnware einem Zuschneideprozess zugeführt In diesem Zuschneideprozess kann der Zuschnitt erstellt werden und insbesondere Falzungen und Schälungen an den Zuschnitt können erfolgen. Dabei kann es so sein, dass nach dem Zerschneiden der Packstoff um 90° gedreht wird, so dass aus einer Kante entlang der Längsrichtung der Bahnware eine Kante entlang der Querrichtung des Zuschnitts wird.

Die Kontaktierung des Funktionselements mit dem elektrischen Elementes erfolgt über eine der Außenkanten des Zuschnitts. Dabei ist zumindest einer der Leiter um eine Schnittkante des Zuschnitts gelegt Eine Schnittkante des Zuschnitts kann entweder ein Längsschnitt oder ein Querschnitt sein. Der Zuschnitt wird entlang der Kanten zu der Packung geformt. Ist einer der Leiter um eine solche Schnittkante geformt, kann ein erster Teil des Leiters im Bereich der Packung auf der Innenseite liegen und ein zweiter auf der anderen Seite der Schnittkante liegender Teil des Leiters auf der Außenseite der Kante. Somit ist es möglich, eine Kontaktierung von innen nach außen zu ermöglichen. Insbesondere kann ein Teil des Leiters auf der ersten Seite der Barriereschicht angeordnet sein und ein Teil des Leiters auf der zweiten Seite der Barriereschicht Somit liegt jeweils ein Teil des Leiters auf einer jeweiligen Seite der Barriereschicht. Auf der Innenseite kann der Leiter beispielsweise auf der inneren Deckschicht angeordnet sein. Auch ist es möglich, dass der Leiter zwischen der Deckschicht und der Barriereschicht angeordnet ist.

Auf der zweiten Seite kann der Leiter beispielsweise auf der Barriereschicht, auf der Trägerschicht oder auf einer äußeren Deckschicht aufgebracht sein. Insbesondere kann der Leiter zwischen der Barriereschicht und der Trägerschicht oder zwischen der Träger und der Deckschicht angeordnet sein.

Der Leiter ist hierbei bevorzugt ein Folienleiter, der von einem Isolationsmaterial umgeben ist. Das die Folie bildende Isolationsmaterial ist insbesondere siegelfähig mit dem Material der Deckschicht. Insbesondere ist das Material das gleiche Material wie das Material der Deckschicht.

Häufig kommt es vor, dass die Trägerschicht entlang einer Längskante geschält wird. Dann wird das Laminat im Bereich der geschälten Längskante umgeschlagen und die umgeschlagene Deckschicht wird mit der Deckschicht der gegenüberliegenden Kante versiegelt. Dann kann es sinnvoll sein, dass zunächst nur ein Teil des Leiters zwischen der Barriereschicht und der inneren Deckschicht bzw. auf der inneren Deckschicht aufgebracht ist. Erst nach dem Umschlagen wird der Leiter dann ausgehend von dem umgeschlagenen Ende nach außen auf die äußere Deckschicht geführt. Die Siegelung entlang der aneinander anliegenden inneren Deckschichten versiegelt dann gleichzeitig den Leiter mit.

Eine besonders einfache Anordnung der Leiter als auch eine einfache Weiterverarbeitung der Leiter kann dann erfolgen, wenn einer der Leiter winklig, insbesondere rechtwinklig zu einer Schnittkante des Zuschnitts verläuft. Der Leiter kann auf einer Deckschicht angeordnet sein, die auf der ersten Seite der Barriereschicht verläuft Im Bereich dieser Deckschicht kann der Leiter im Bereich einer Schnittkante des Zuschnitts verlaufen.

Insbesondere im Bereich der Schnittkante erfolgt eine anschließende Versiegelung entweder über eine Schälung und ein anschließendes Umschlagen und Versiegeln der Deckschichten übereinander oder ein überlappendes Übereinanderlegen der Längskanten und Verwendung eines Siegelstreifens. In beiden Fällen ist jedoch im Bereich der Schnittkante die Barriereschicht doppelt, so dass ein Durchbrechen der Barriereschicht im Bereich der Schnittkante, insbesondere im Bereich der späteren Siegelnaht unproblematisch hinsichtlich der Diffusion durch die Barriereschicht ist Einerseits liegt die Barriereschicht dort nicht mehr unmittelbar an dem Produkt an, andererseits ist die Barriereschicht dort doppelt gefasst

Der Zuschnitt kann eine Längs- und eine Querkante aufweisen und der Leiter verläuft vorzugsweise parallel zur Längskante.

Ein Leiter kann aus zwei Teilen gebildet sein, wobei ein erstes Teil als festes Ende fest auf einer Schicht der ersten Seite der Barriereschicht angeordnet sein kann. Das feste Ende kann unmittelbar auf der Barriereschicht, zwischen der Barriereschicht und der Deckschicht oder auf der Deckschicht angeordnet sein. Dann kann im Zuschnitt ein freies Ende vorhanden sein, welches erst nach dem Formen der Packung auf einer äußeren Deckschicht befestigt werden kann. Dazu ist es notwendig, dass das freie Ende des Leiters über eine Schnittkante hinausragt. Das freie Ende ragt, nachdem die Packung geformt wurde, über eine Naht der Packung hinaus. Dieses freie Ende kann dann dazu genutzt werden, eine Kontaktierung mit dem elektrischen Element vorzunehmen.

Das freie Ende kann entweder über eine Längsnaht oder eine Quernaht hinausragen. Ragt das freie Ende über eine Längsnaht hinaus, so ragt es an der Verpackung entweder bodenseitig oder deckelseitig über eine Naht hinaus. Liegt das freie Ende über der Querkante, so ragt es im Bereich einer Längsnaht der Packung hinaus.

Wie bereits erläutert, kann die Barriereschicht in zwei Teile durchtrennt sein. Im Bereich eines Zuschnitts erfolgt die Durchtrennung entlang einer Richtung durchgehend. Das bedeutet, dass in einem Zuschnitt zwei elektrisch voneinander isolierte Bereiche der Barriereschicht vorliegen. Jeder der Bereiche kann zumindest einen Teil des elektrischen Leiters bilden.

Es ist bevorzugt, dass die Durchtrennung der Barriereschicht nicht in einem Bereich des Zuschnitts liegt, der in der Verpackung unmittelbar an dem zu verpackenden Produkt anliegen kann. Daher bietet es sich an, dass die Trennung zwischen den Bereichen im Bereich einer Längskante oder einer Querkante, insbesondere parallel zu einer Längskante oder einer Querkante verläuft Im Bereich einer Kante erfolgt ein Verschließen des Zuschnitts zu der Packung. Dort liegen in der Regel zwei Kanten übereinander. Dies kann ein einfacher Überlappstoß sein oder es ist möglich, dass eine Kante umgeschlagen ist und der Überlapp ausschließlich im Bereich des umgeschlagenen Teils liegt Im Falle des Umschlagens der Kante kann eine Schälung zumindest von Teilen der Trägerschicht sinnvoll sein, um die Materialstärke des Überlappungsbereiches in Grenzen zu halten.

Im Bereich der überlappenden Kanten erfolgt eine Siegelung. Durch die Überlappung im Bereich der Kanten kommt es dazu, dass zwei Barriereschichten übereinander liegen. Zusätzlich liegt die Barriereschicht im Bereich der Überlappung häufig außerhalb des Bereiches der Packung, der mit dem Produkt in Kontakt steht Beim einfachen Überlappstoß ist zumindest die außenliegende Kante mit ihrer Barriereschicht außerhalb des Bereiches, der mit dem Produkt in Verbindung steht Bei einem Umschlagen, insbesondere mit Schälung, liegen die aufeinanderliegenden Kanten jeweils so, dass sie nicht in Kontakt mit dem Produkt sind bzw. in dem Bereich liegen, der mit dem Produkt in Kontakt gerät Insbesondere erfolgt die Trennung in dem Bereich der Kante, der in der Verpackung einen Überlappungsbereich der Kanten bildet. Vorzugsweise liegt die Trennung im Bereich der Siegelnaht der Kante.

Wie bereits erläutert, kann die Barriereschicht eine Ausnehmung aufweisen. Auch wurde bereits erläutert, dass zumindest einer der elektrischen Leiter durch die Ausnehmung geführt ist. Es wird nunmehr auch vorgeschlagen, dass die Ausnehmung im Bereich einer Längskante oder einer Querkante ist Die Ausnehmung ist vorzugsweise in einem Bereich, wie er zuvor bevorzugt für die Trennung zwischen den beiden Bereichen der Barriereschicht beschrieben wurde. Dabei ist die Ausnehmung insbesondere im Bereich einer Längskante oder einer Querkante, insbesondere im Bereich einer Schälung der Längskante und/oder der Querkante. Auch ist die Ausnehmung bevorzugt im Bereich einer Siegelnaht der Längskante und/oder der Querkante.

Wenn der Zuschnitt gefaltet wird und zumindest entlang einer Längskante eine Siegelung vorgenommen wurde, kann man von einem Packungsmantel sprechen.

Wird anschließend noch der Boden versiegelt, so entsteht eine Packung, in die ein zu verpackendes Gut gefüllt werden kann. Das Funktionselement ist vorzugsweise im Bereich einer unteren Hälfte, insbesondere im Bereich eines unteren Drittels der Packung angeordnet, insbesondere im Bereich des Bodens. Dies stellt sicher, dass dieses Funktionselement den Zustand des Produktes auch bei zumindest in Teilen geleerter Verpackung erfassen kann. Das verpackte Gut verbleibt im Bereich des Bodens, so dass eine sensorische Erfassung der Eigenschaften des verpackten Gutes vorzugsweise im Bereich des Bodens erfolgt Das Funktionselement kann entweder an einer Mantelfläche der Packung im Bereich des Bodens sein, oder unmittelbar am Boden angeordnet sein.

In der Verpackung verläuft der Leiter vorzugsweise entlang einer Längsnaht oder einer Quernaht

Gemäß einem Ausführungsbeispiel wird vorgeschlagen, dass im Bereich der ersten Seite der Barriereschicht eine siegelfähige Deckschicht vorgesehen ist, dass die siegelfähige Deckschicht im Bereich zumindest einer Naht gesiegelt ist, wobei zumindest einer der Leiter im Bereich der entstandenen Siegelnaht verläuft.

Insbesondere im Bereich eines Überlappstoßes ohne eine umgeschlagene Kante kann die Versiegelung entlang einer Längskante und/oder einer Querkante mit einem Siegelstreifen erfolgen. Da im Bereich eines einfachen Überlappstoßes nicht mehr die inneren Deckschichten unmittelbar aufeinanderliegen, kann ein Versiegeln unmittelbar der Deckschichten unmöglich sein, beispielsweise aufgrund von Farbaufträgen. Aus diesem Grunde ist eine sichere Versiegelung mit einem Siegelstreifen notwendig. Es ist vorgeschlagen, dass der Leiter in dem Siegelstreifen selbst angeordnet ist. Der Siegelstreifen kann dann beispielsweise über eine Querkante und/oder eine Längskante hinausragen und nach dem Versiegeln umgeschlagen werden. Somit wird durch den Siegelstreifen der Leiter vom Inneren der Packung zum Äußeren der Packung geführt

Das zuvor beschriebene Durchbrechungselement schwächt die Barriereschicht. Um die damit einhergehende Gefahr der Kontaminierung des Produktes zu minimieren bzw. auszuschließen, ist das Durchbrechungselement bevorzugt im Bereich der Siegelnaht angeordnet. Das Durchbrechungselement kann so wie oben für den Leiter beschrieben angeordnet sein.

Auch die Trennung der Barriereschicht in zwei Bereiche erfolgt vorzugsweise im Bereich der Siegelnaht aus denselben Gründen.

Auch eine Ausnehmung kann in der Barriereschicht vorgesehen sein, wie beschrieben. Auch wird vorgeschlagen, dass diese Ausnehmung im Bereich der Siegelnaht angeordnet ist Eine Siegelnaht kann entweder durch einen Siegelstreifen gebildet sein, oder durch eine unmittelbare Versiegelung einer inneren Deckschicht einer äußeren Deckschicht oder durch eine Versiegelung einer umgeschlagenen inneren Deckschicht mit einer darauf aufliegenden inneren Deckschicht In allen Fällen kann dann die Schwächung der Barriereschicht in dem Bereich sein, der nicht unmittelbar im Kontakt mit dem Produkt ist

Wie bereits erläutert, kann das Funktionselement zumindest einen Sensor und/oder einen Speicher aufweisen. Auch das elektrische Element kann ein Speicher aufweisen. Darüber hinaus kann das elektrische Element eine Sende- und/oder Empfangseinheit aufweisen. Insbesondere kann dies eine Sende- und/oder Empfangseinheit für Nahfunk, insbesondere für NFC Technologie oder RFID Technologie aufweisen. Dazu kann das elektrische Element energetisch eigengespeist sein, beispielsweise durch eine Batterie oder einen Kondensator. Auch ist es möglich und besonders bevorzugt, wenn das Funktionselement und/oder elektrische Element energetisch fremdgespeist ist Diese energetische Fremdspeisung kann durch ein magnetisches oder elektrisches Feld einer Ausleseeinheit oder einer Schreibeinheit erfolgen. Insbesondere kann eine elektromagnetische Kopplung einer in dem elektrischen Element gebildeten Empfangsspule mit einer Sendespule die energetische Speisung bewirken. Durch eine Sendespule kann in der Empfangsspule ein Strom induziert werden, der zum Betreiben der ersten elektronischen Einheit und/oder der zweiten elektronischen Einheit dienen kann. So kann es beispielsweise sinnvoll sein, durch das Erregerfeld den Sensor zu aktivieren und im Falle der Aktivierung zumindest einen Sensormesswert zu erfassen und abzuspeichern. Wird das Erregerfeld wieder abgeschaltet, verbleibt zumindest der gespeicherte Messwert in dem Speicher. Ein solcher Messwert kann unmittelbar oder zu einem späteren Zeitpunkt, bei einer erneuten Erregung, ausgelesen werden, so dass zumindest ein historischer und/oder ein aktueller Sensormesswert ausgelesen werden kann. Da die Elemente in dem Packstoff möglichst einfach gestaltet sein müssen, ist bevorzugt, wenn diese lediglich zum Erfassen der Messergebnisse und Abspeichern zumindest eines Messergebnisses sowie zum Aussenden eines solchen Messergebnisses eingerichtet sind. Eine anschließende Auswertung der Messergebnisse kann in einer Auslesevorrichtung erfolgen.

Die Auslesevorrichtung kann mobiler Computer insbesondere ein Mobiltelefon, ein Smartphone, ein Personal Digital Assistent oder dergleichen sein. In diesem kann eine Applikation vorhanden sein, die zur Auswertung der Messergebnisse eingerichtet ist. Eine solche Applikation kann eigenständig erfinderisch sein. Die Applikation aktiviert die Sendespule zum energetischen Speisen der ersten und zweiten elektronischen Einheit Über die Sendespule kann beispielsweise über eine Beeinflussung des Wechselflusses eine Information von dem elektrischen Element empfangen werden. Auch kann das elektrische Element beispielsweise in einem anderen Frequenzbereich als der des Erregerfeldes die Informationen aussenden und die Sendespule oder eine weitere Spule kann diese Information empfangen. Mit Hilfe der Applikation können Messwerte ausgewertet werden.

Der Vollständigkeit halber sei nachfolgend der Aufbau der Schichten des Packungslaminats (Packstoffs) näher beschrieben.

Die Schichten der Schichtfolge sind miteinander verbunden. Der in dieser Beschreibung verwendete Begriff "verbunden" bzw. "Verbund" umfasst die über Van-der-Waals Anziehungskräfte hinausgehende Haftung zweier Gegenstande. Sofern nicht anders angegeben können in der Schichtfolge die Schichten mittelbar, das heißt mit einer oder mindestens zwei Zwischenschichten, oder unmittelbar, das heißt ohne Zwischenschicht, aufeinander folgen. Für den flächenförmigen Verbund bedeutet dies beispielsweise, dass die Barriereschicht direkt und damit unmittelbar mit der ersten Polyolefinschicht oder auch indirekt über eine Haftvermittlerschicht verbunden sein kann. Weiterhin kann auch die weitere Polyolefinschicht direkt und unmittelbar mit der Trägerschicht verbunden sein, es können sich jedoch auch weitere Gegenstande, beispielsweise in Form von weiteren Polymerschichten dazwischen befinden, wobei eine unmittelbare Verbindung bevorzugt ist. Die Formulierung "beinhaltend eine Schichtfolge", wie sie vorstehend verwendet wird, bedeutet, dass in dem erfindungsgemäßen Verbund zumindest die angegebenen Schichten in der angegebenen Reihenfolge vorliegen können. Diese Formulierung besagt nicht zwingend, dass diese Schichten unmittelbar aufeinander folgen.

Eine erste Polyolefinschicht kann als Deckschicht die Barriereschicht auf einer der Trägerschicht abgewandten Seite überlagern. Eine weitere Polyolefinschicht kann als Außenschicht die Trägerschicht auf einer der Barriereschicht abgewandten Seite überlagert Bevorzugt grenzt die weitere Polyolefinschicht an die Trägerschicht an.

Die erste Polyolefinschicht sowohl wie die weitere Polyolefinschicht, wie auch alle weiteren Polymerschichten können weitere Bestandteile aufweisen. Diese Polyolefinschichten werden bevorzugt in einem Extrudierverfahren in das flächenförmige Verbundmaterial ein- bzw. aufgebracht. Die weiteren Bestandteile der Polyolefinschichten sind bevorzugt Bestandteile, die das Verhalten der Polymerschmelze beim Auftragen als Schicht nicht nachteilig beeinflussen. Die weiteren Bestandteile können beispielsweise anorganische Verbindungen, wie Metallsalze oder weitere Kunststoffe, wie weitere thermoplastische Kunststoffe sein. Es ist jedoch auch denkbar, dass die weiteren Bestandteile Füllstoffe oder Pigmente sind, beispielsweise Ruß oder Metalloxide.

Als geeignete thermoplastische Kunststoffe kommen für die weiteren Bestandteile insbesondere solche in Betracht, die durch ein gutes Extrusionsverhalten leicht verarbeitbar sind. Hierunter eignen sich durch Kettenpolymerisation erhaltene Polymere, insbesondere Polyester oder Polyolefine, wobei cyclische Olefin-Copolymere (COC), polycyclische Olefin-Copolymere (POC), insbesondere Polyethylen und Polypropylen, besonders bevorzugt sind und Polyethylen ganz besonders bevorzugt ist Unter den Polyethylenen sind HDPE, MDPE, LDPE, LLDPE, VLDPE und PE sowie Mischungen aus mindestens zwei davon bevorzugt. Es können auch Mischungen aus mindestens zwei thermoplastischen Kunststoffen eingesetzt werden.

Geeignete Polyolefinschichten besitzen eine Schmelzflussrate (MFR - melt flow rate) in einem Bereich von 1 bis 25 g/10 min, vorzugsweise in einem Bereich von 2 bis 20 g/10 min und besonders bevorzugt in einem Bereich von 2,5 bis 15 g/10 min, und eine Dichte in einem Bereich von 0,890 g/cm³ bis 0,980 g/cm³, vorzugsweise in einem Bereich von 0,895 g/cm³ bis 0,975 g/cm³, und weiter bevorzugt in einem Bereich von 0,900 g/cm³ bis 0,970 g/cm³. Die Polyolefinschichten besitzen bevorzugt mindestens eine Schmelztemperatur in einem Bereich von 80 bis 155°C, vorzugsweise in einem Bereich von 90 bis 145°C und besonders bevorzugt in einem Bereich von 95 bis 135°C. Bevorzugt beinhaltet der flächenförmige Verbund zwischen der Barriereschicht und der Trägerschicht eine Polyolefinschicht, bevorzugt eine Polyethylenschicht. Weiter bevorzugt beinhaltet der Verbundvorläufer zwischen der Barriereschicht und der Trägerschicht eine Polyolefinschicht, bevorzugt eine Polyethylenschicht.

Als Trägerschicht kann jedes dem Fachmann für diesen Zweck geeignete Material eingesetzt werden, welches eine ausreichende Festigkeit und Steifigkeit aufweist, um den Behälter soweit Stabilität zu geben, dass der Behälter im gefüllten Zustand seine Form im Wesentlichen beibehält. Neben einer Reihe von Kunststoffen sind auf Pflanzen basierende Faserstoffe, insbesondere Zellstoffe, vorzugsweise verleimte, gebleichte und/oder ungebleichte Zellstoffe bevorzugt, wobei Papier und Karton besonders bevorzugt sind. Das Flächengewicht der Trägerschicht liegt vorzugsweise in einem Bereich von 120 bis 450 g/m², besonders bevorzugt in einem Bereich von 130 bis 400 g/m² und am meisten bevorzugt in einem Bereich von 150 bis 380 g/m². Ein bevorzugter Karton weist in der Regel einen ein- oder mehrschichtigen Aufbau auf und kann ein- oder beidseitig mit einer oder auch mehreren Deckschichten beschichtet sein. Weiterhin besitzt ein bevorzugter Karton eine Restfeuchtigkeit von weniger als 20 Gew.-%, bevorzugt von 2 bis 15 Gew.-% und besonders bevorzugt von 4 bis 10 Gew.-% bezogen auf das Gesamtgewicht des Kartons. Ein besonders bevorzugter Karton weist einen mehrschichtigen Aufbau auf. Weiterhin bevorzugt besitzt der Karton auf der zur Umgebung hin weisenden Oberfläche mindestens eine, besonders bevorzugt jedoch mindestens zwei Lagen einer Deckschicht, die dem Fachmann als "Strich" bekannt ist

Als "Strich" werden in der Papierherstellung meist anorganische Feststoffpartikel beinhaltende flüssige Phasen, vorzugsweise Kreide-, Gips- oder Ton-haltige Lösungen, bezeichnet, welche auf die Oberfläche des Karton aufgebracht werden. Weiterhin besitzt ein bevorzugter Karton ein Scott-Bond Wert in einem Bereich von 100 bis 360 J/m², bevorzugt von 120 bis 350 J/m² und insbesondere bevorzugt von 135 bis 310 J/m². Durch die vorstehend genannten Bereiche gelingt es, einen Verbund bereit zu stellen, aus dem sich ein Behälter mit hoher Dichtigkeit, leicht und in geringen Toleranzen falten lässt.

Die Barriereschicht ist eine Metallschicht. Als Metallschicht eignen sich prinzipiell alle Schichten mit Metallen, die dem Fachmann bekannt sind und eine hohe Licht-, und Sauerstoffundurchlässigkeit schaffen können. Gemäß einer bevorzugten Ausführungsform kann die Metallschicht als Folie oder als abgeschiedene Schicht vorliegen, z. B. nach einer physikalischen Gasphasenabscheidung. Die Metallschicht ist vorzugsweise eine ununterbrochene Schicht. Gemäß einer weiteren bevorzugten Ausführungsform weist die Metallschicht eine Dicke in einem Bereich von 3 bis 20 µm, bevorzugt in einem Bereich von 3,5 bis 12 µm und besonders bevorzugt in einem Bereich von 4 bis 10 µm auf.

Bevorzugt ausgewählte Metalle sind Aluminium, Eisen oder Kupfer. Als Eisenschicht kann eine Stahlschicht, z. B. in Form einer Folie bevorzugt sein. Weiterhin bevorzugt stellt die Metallschicht eine Schicht mit Aluminium dar. Die Aluminiumschicht kann zweckmäßig aus einer Aluminiumlegierung, beispielsweise AlFeMn, AlFe1,5Mn, AlFeSi oder AlFeSiMn bestehen. Die Reinheit liegt üblicherweise bei 97,5% und höher, vorzugsweise bei 98,5% und höher, jeweils bezogen auf die gesamte Aluminiumschicht. In einer besonderen Ausgestaltung, besteht die Metallschicht aus einer Aluminiumfolie. Geeignete Aluminiumfolien besitzen eine Dehnbarkeit von mehr als 1%, bevorzugt von mehr als 1,3% und besonders bevorzugt von mehr als 1,5%, und eine Zugfestigkeit von mehr als 30 N/mm², bevorzugt mehr als 40 N/mm² und besonders bevorzugt mehr als 50 N/mm². Geeignete Aluminiumfolien zeigen im Pipettentest eine Tropfengröße von mehr als 3 mm, bevorzugt mehr als 4 mm und besonders bevorzugt von mehr als 5 mm. Geeignete Legierungen zum Erstellen von Aluminiumschichten oder -folien sind unter den Bezeichnungen EN AW 1200, EN AW 8079 oder EN AW 8111 erhältlich.

Im Falle einer Metallfolie als Barriereschicht kann ein- und/oder beidseitig der Metallfolie eine Haftvermittlerschicht zwischen der Metallfolie und einer nächstgelegenen Polymerschicht vorgesehen sein. Gemäß einer besonderen Ausgestaltung des erfindungsgemäßen Behälters ist jedoch auf keiner Seite der Metallfolie, zwischen der Metallfolie und der nächstgelegenen Polymerschicht, eine Haftvermittlerschicht vorgesehen.

Weiterhin bevorzugt kann als Barriereschicht eine Metalloxidschicht sein. Als Metalloxidschichten kommen alle Metalloxidschichten in Betracht, die dem Fachmann geläufig sind und geeignet erscheinen, um eine Barrierewirkung gegenüber Licht, Dampf und/oder Gas zu erzielen. Insbesondere bevorzugt sind Metalloxidschichten basierend auf den schon zuvor genannten Metallen Aluminium, Eisen oder Kupfer, sowie solche Metalloxidschichten, die auf Titan- oder Siliziumoxidverbindungen basieren. Eine Metalloxidschicht wird beispielhaft durch Bedampfen einer Kunststoffschicht, beispielsweise eine orientierte Polypropylenfolie mit Metalloxid erzeugt Ein bevorzugtes Verfahren hierfür ist die physikalische Gasphasenabscheidung.

Gemäß einer weiteren bevorzugten Ausführungsform kann die Metallschicht der Metalloxidschicht als Schichtenverbund aus einer oder mehrerer Kunststoffschichten mit einer Metallschicht vorliegen. Eine solche Schicht ist zum Beispiel erhältlich durch Bedampfen einer Kunststoffschicht, beispielsweise eine orientierte Polypropylenfolie, mit Metall. Ein bevorzugtes Verfahren hierfür ist die physikalische Gasphasenabscheidung.

Um die Öffenbarkeit des erfindungsgemäßen Behälters bzw. des flächenförmigen Verbundes zu erleichtern, kann die Trägerschicht mindestens eine Ausnehmung (synonym: Loch, Öffnung) aufweisen. In einer besonderen Ausgestaltung ist die Ausnehmung mindestens mit der Barriereschicht und mindestens der ersten Polyolefinschicht als Deckschichten überdeckt Bevorzugt ist ein flächenförmiger Verbund, wobei die Trägerschicht mindestens ein Loch aufweist, das mindestens mit der Barriereschicht und mindestens mit der ersten Polyolefinschicht, und der weiteren Polyolefinschicht überdeckt ist. Ferner können zwischen den bereits genannten Schichten eine oder mehrere weitere Schichten, insbesondere Haftvermittlerschichten, vorgesehen sein. Hierbei ist es bevorzugt, dass die Lochdeckschichten mindestens teilweise, vorzugsweise zu mindestens 30%, bevorzugt mindestens 70% und besonders bevorzugt zu mindestens 90% der durch das Loch gebildeten Fläche miteinander verbunden sind.

Gemäß einer besonderen Ausgestaltung ist es bevorzugt, dass das Loch den gesamten Verbund durchdringt und durch einen das Loch verschließenden Verschluss bzw. Öffnungsvorrichtung überdeckt wird. Im Zusammenhang mit einer ersten bevorzugten Ausführungsform kann das in der Trägerschicht vorgesehene Loch jede dem Fachmann bekannte und für verschiedene Verschlüsse, Trinkhalme oder Öffnungshilfen geeignete Form haben. Meist wird die Öffnung eines flächigen Verbundes oder eines Behälters mit einem flächigen Verbund durch mindestens teilweises Zerstören der das Loch überdeckenden Lochdeckschichten erzeugt Dieses Zerstören kann durch Schneiden, Eindrücken in den Behälter oder Herausziehen aus dem Behälter erfolgen. Das Zerstören kann durch ein mit dem Behälter verbundenen und im Bereich des Lochs, meist oberhalb des Lochs angeordneten, öffenbaren Verschluss oder einen Trinkhalm, der durch die das Loch bedeckenden Lochdeckschichten gestoßen wird, erfolgen.

Gemäß einer weiteren bevorzugten Ausführungsform weist die Trägerschicht des Verbunds eine Vielzahl von Löchern in Form einer Perforation auf, wobei die einzelnen Löcher mindestens mit der Barriereschicht und einer der ersten Polyolefinschicht als Lochdeckschichten überdeckt sind. Ein aus einem solchen Verbund hergestellter Behälter kann dann durch Aufreißen entlang der Perforation geöffnet werden. Derartige Löcher für Perforationen werden bevorzugt mittels eines Lasers erzeugt Besonders bevorzugt ist der Einsatz von Laserstrahlen, wenn eine Metallfolie oder eine metallisierte Folie als Barriereschicht eingesetzt wird. Es ist ferner möglich, dass die Perforation durch mechanische, meist Klingen aufweisende, Perforationswerkzeuge eingebracht wird.

Als Haftvermittler in der Haftvermittlerschicht kommen alle Kunststoffe in Betracht, die durch Funktionalisierung mittels geeigneter funktioneller Gruppen geeignet sind, durch das Ausbilden von Ionenbindungen oder kovalenten Bindungen zu der Oberfläche der jeweils anderen Schicht eine feste Verbindung zu erzeugen. Vorzugsweise handelt es sich um funktionalisierte Polyolefine, die durch Co-Polymerisation von Ethylen mit Acrylsäuren wie Acrylsäure, Methacrylsäure, Crotonsäure, Acrylaten, Acrylatderivaten oder Doppelbindungen tragenden Carbonsäureanhydriden, beispielsweise Maleinsäureanhydrid, oder mindestens zwei davon, erhalten wurden. Hierunter sind Polyethylen-maleinsäureanhydrid-Pfropfpolymere (EMAH), Ethylen-Acrylsäure-Copolymere (EAA) oder Ethylen-Methacrylsäure-Copolymere (EMAA) bevorzugt, welche beispielsweise unter den Handelsbezeichnungen Bynel® und Nucrel® durch DuPont oder Escor® durch ExxonMobile Chemicals vertrieben werden.

Ein bevorzugtes Polyolefin ist ein Polyethylen oder ein Polypropylen oder beides. Ein bevorzugtes Polyethylen ist eines ausgewählt ist aus der Gruppe bestehend aus einem LDPE, einem LLDPE, und einem HDPE, oder eine Kombination aus mindestens zwei davon. Ein weiteres bevorzugtes Polyolefin ist ein mPolyolefin. Geeignete Polyethylene besitzen eine Schmelzflussrate (MFR - melt flow rate) in einem Bereich von 1 bis 25 g/10 min, vorzugsweise in einem Bereich von 2 bis 20 g/10 min und besonders bevorzugt in einem Bereich von 2,5 bis 15 g/10 min, und eine Dichte in einem Bereich von 0,910 g/cm³ bis 0,935 g/cm³, vorzugsweise in einem Bereich von 0,912 g/cm² bis 0,932 g/cm³, und weiter bevorzugt in einem Bereich von 0,915 g/cm³ bis 0,930 g/cm³.

Ein mPolyolefin ist ein Polyolefin, welches mittels eines Metallocen-Katalysators hergestellt wurde. Ein Metallocen ist eine Metallorganische Verbindung, in welchem ein zentrales Metallatom zwischen zwei organischen Liganden, wie beispielsweise Cyclopentadienyl-Liganden angeordnet ist Ein bevorzugtes mPolyolefin ist ein mPolyethylen oder ein mPolypropylen oder beides. Ein bevorzugtes mPolyethylen ist eines ausgewählt ist aus der Gruppe bestehend aus einem mLDPE, einem mLLDPE, und einem mHDPE, oder eine Kombination aus mindestens zwei davon.

Eine bevorzugte Farbschicht beinhaltet Farbmittel zu einem Anteil in einem Bereich von 5 bis 15 Gew.-%, bevorzugt von 8 bis 15 Gew.-%, bevorzugter von 13 bis 15 Gew.-%, bezogen auf die Farbschicht. Eine weitere bevorzugte Farbschicht beinhaltet ferner ein Anwendungsmedium. Ein bevorzugtes Anwendungsmedium ist ein organisches Medium. Ein bevorzugtes organisches Medium ist ein organisches Bindemittel. Ein bevorzugtes organisches Bindemittel ist ein Thermoplast Ein bevorzugter Thermoplast ist Polyvinylbutyral (PVB). Bevorzugt grenzt die Farbschicht an die weitere Polyolefinschicht an, wobei die weitere Polyolefinschicht bevorzugt an die Trägerschicht angrenzt Bevorzugt ist Farbschicht erhältlich durch ein Drucken. Ein bevorzugtes Drucken ist hierbei ein Offsetdrucken oder ein Tiefdrucken oder beides. Eine weitere bevorzugte Farbschicht ist auf einer der Trägerschicht abgewandten Seite der Farbschicht durch keine weitere Schicht der Schichtfolge überlagert

Ein bevorzugtes Farbmittel ist ein farbgebender Stoff nach der Norm DIN 55943 . Ein weiteres bevorzugtes Farbmittel ist ein Pigment oder eine Farbstoff oder beides. Ein besonders bevorzugtes Farbmittel ist ein Pigment. Ein weiteres bevorzugtes Farbmittel ist ein natürliches Farbmittel oder ein synthetisches Farbmittel oder beides. Ein weiteres bevorzugtes Farbmittel ist eines ausgewählt aus der Gruppe bestehend aus einem weißen Farbmittel, einem schwarzen Farbmittel, und einem bunten Farbmittel, oder eine Kombination aus Mindestens zwei davon. Ein weiteres bevorzugtes Farbmittel ist eine Effektfarbmittel oder ein Leuchtfarbmittel oder beides.

Die Merkmale der Verfahren und Vorrichtungen sind frei miteinander kombinierbar. Insbesondere können Merkmale und Teilmerkmale der Beschreibung und/oder der abhängigen sowie unabhängigen Ansprüche, auch unter vollständiger oder teilweiser Umgehung von Merkmalen oder Teilmerkmalen der unabhängigen Ansprüche, in Alleinstellung oder frei miteinander kombiniert eigenständig erfinderisch sein.

Nachfolgend wird der Gegenstand anhand einer Ausführungsbeispiele zeigenden Zeichnung näher erläutert In der Zeichnung zeigen:
- Fig. 1: eine schematische Ansicht eines ersten Funktionselements in dem Packstoff;
- Fig. 2: eine schematische Ansicht eines ersten und eines elektrischen Elements in einem Packstoff;
- Fig. 3: eine schematische Ansicht einer Durchführung eines Leiters durch eine Barriereschicht;
- Fig. 4: eine schematische Ansicht einer Durchführung eines Leiters durch eine Barriereschicht;
- Fig. 5: einen Zuschnitt mit potentiellen Positionen von Leitungen;
- Fig. 6a: einen geöffneten Packungsmantel;
- Fig. 6b: einen geschlossenen Packungsmantel;
- Fig. 7a: einen Packungsmantel mit einem Siegelstreifen;
- Fig. 7b: eine Ansicht eines Siegelstreifens;
- Fig. 8a: ein Ausführungsbeispiel einer Kontaktierung eines Leiters in einer Siegelnaht;
- Fig. 8b: mögliche Durchbrechungen in der Barriereschicht;
- Fig. 8c: eine Ausgestaltung eines Durchbrechungselements;
- Fig. 8d: eine Ausgestaltung eines Durchbrechungselements;
- Fig. 8e: eine Möglichkeit einer Kontaktierung;
- Fig. 9a: eine Kontaktierung entlang des Schichtaufbaus;
- Fig. 9b: eine Anordnung von Leitern und Elementen bei einem Schichtaufbau;
- Fig. 9c: eine mögliche Durchführung eines Leiters bei einem Schichtaufbau;
- Fig. 9d: eine weitere Möglichkeit einer Kontaktierung bei einem Schichtaufbau;
- Fig. 9e: eine weitere Möglichkeit einer Anordnung in einem Schichtaufbau;
- Fig. 10a-c: mögliche Anordnungen von Durchbrechungselementen;
- Fig. 11a: eine schematische Ansicht eines Sendeelementes;
- Fig. 11b: eine schematische Ansicht einer Anordnung von Sendelementen auf einem Endlosband;

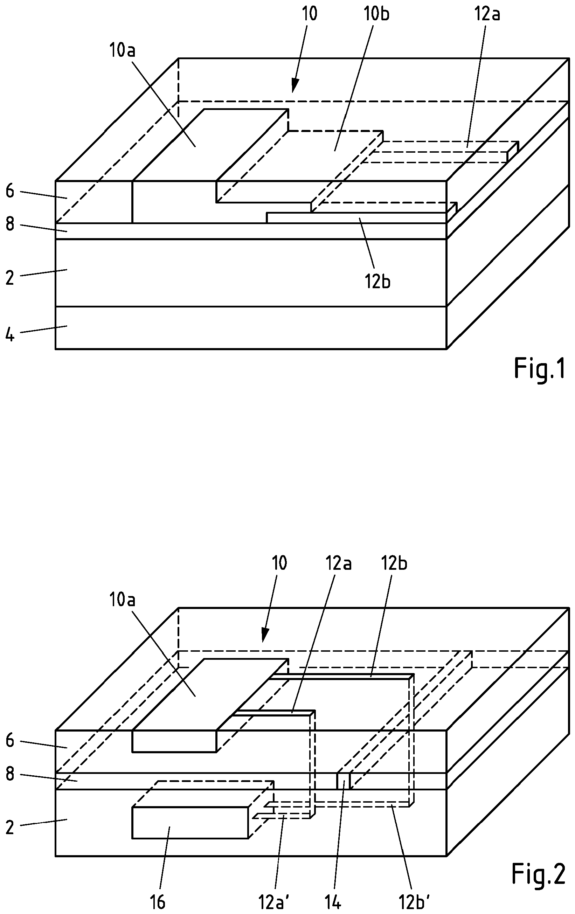

Fig. 1 zeigt eine schematische Ansicht eines Schichtaufbaus eines Packstofflaminats (Packstoffs). Der Schichtaufbau kann eine Trägerschicht 2, eine äußere Deckschicht 4, eine innere Deckschicht 6 sowie eine Barriereschicht 8 aufweisen. Die Trägerschicht 2 ist vorzugsweise aus einem Trägermaterial, wie beispielsweise Karton. Die Deckschichten 4, 6 sind insbesondere aus Kunststoff, beispielsweise PE und die Barriereschicht 8 ist insbesondere metallisch, beispielsweise aus Aluminium. Zu weiteren Details der Schichten wird auf die obigen Ausführungen verwiesen. Es versteht sich, dass dieser Schichtenaufbau rein beispielhaft ist.

In der Fig. 1 ist zu erkennen, dass die Deckschicht 6 beispielsweise einen integrierten Sensor 10 aufweist Der Sensor 10 hat einen Bereich 10a, der die Deckschicht 6 durchbricht und somit an der Oberfläche der Deckschicht liegt Der Sensor 10 kann jedoch auch einen Bereich 10b aufweisen, der von der Deckschicht 6 bedeckt ist Auch ist es möglich, dass ausschließlich ein Bereich 10a oder ein Bereich 10b des Sensors 10 vorgesehen ist Im Bereich 10a ist eine unmittelbare Messung am Produkt möglich und im Bereich 10b ist eine mittelbare Messung am Produkt möglich.

Wie zu erkennen ist, ist der Sensor 10 auf der Barriereschicht 8 aufgebracht Dazu ist der Sensor 10 mit einem Isolator gegenüber der Barriereschicht isoliert Ausgehend von dem Sensor 10 erstrecken sich zwei Leitungen 12a, b entlang der Barriereschicht 8. Die Leitungen 12a, b können dabei insbesondere Folienleiter sein, die isoliert sind und isoliert auf der Barriereschicht 8 aufliegen.

Zur Kontaktierung des Sensors 10 mit einem elektrischen Element jenseits der Barriereschicht 8 ist es notwendig, die Barriereschicht 8 zu durchdringen. Gleichzeitig müssen die beiden Leiter 12a, b voneinander elektrisch isoliert bleiben, um eine Signalübertragung zu ermöglichen. Eine solche Kontaktierung ist beispielhaft in der Fig. 2 gezeigt.

Auch in der Fig. 2 ist eine Trägerschicht 2, eine Barriereschicht 8 sowie eine Deckschicht 6 gezeigt Ferner ist ein Sensor 10 vorgesehen, der die Deckschicht 6 in einem Bereich 10a durchbricht

Anders als in der Fig. 1 sind die Leiter 12a, 12b jedoch nicht als Folienleiter, sondern beispielsweise als gedruckte Leitungen aus der Deckschicht 8 gebildet Ferner ist in der Fig. 2 zu erkennen, dass die Barriereschicht an einer Trennstelle 14 aufgetrennt ist. Die Trennstelle 14 verläuft dabei vorzugsweise parallel zu einer Quer- oder Längsnaht und erstreckt sich vorzugsweise über einen gesamten Zuschnitt von einer Oberkante zu einer Unterkante oder zwischen zwei Seitenkanten. Dadurch wird gewährleitet, dass die Barriereschicht 8 in zwei Bereiche aufgeteilt sind, die elektrisch durch die Trennstelle 14 voneinander isoliert sind. Hierdurch ist es möglich, den Leiter 12a mit einem ersten Teil der Barriereschicht 8 zu kontaktieren und den Leiter 12 b mit einem zweiten Bereich. Diese Kontaktierung kann beispielsweise mit Hilfe von Stiften erfolgen, die die Deckschicht 6 durchbrechen.

Auf der anderen Seite der Barriereschicht 8, im Bereich der Trägerschicht 2, kann ebenfalls eine Kontaktierung der beiden Bereiche der Barriereschicht 8 vorgenommen werden und der Leiter 12a wird durch den Leiter 12a' fortgeführt und der Leiter 12b durch den Leiter 12b'. Der Leiter 12a' ist elektrisch mit dem Leiter 12a kurzgeschlossen und der Leiter 12b' ist elektrisch mit dem Leiter 12b kurzgeschlossen. Zwischen den Leitern 12a, b ist eine elektrische Isolation, beispielsweise durch die Trennstelle 14 in der Barriereschicht 8 vorgesehen.

Die Leiter 12a', 12b' kontaktieren ein elektrisches Element, beispielsweise einen Transmitter 16, welcher mit einem Prozessor, einem Speicher und einer Antenne ausgestattet sein kann.

Der Transmitter 16 kann beispielsweise Messwerte des Sensors 10 über die Leiter 12a, a', 12b, b' auslesen und über eine Antenne drahtlos zur Verfügung stellen.

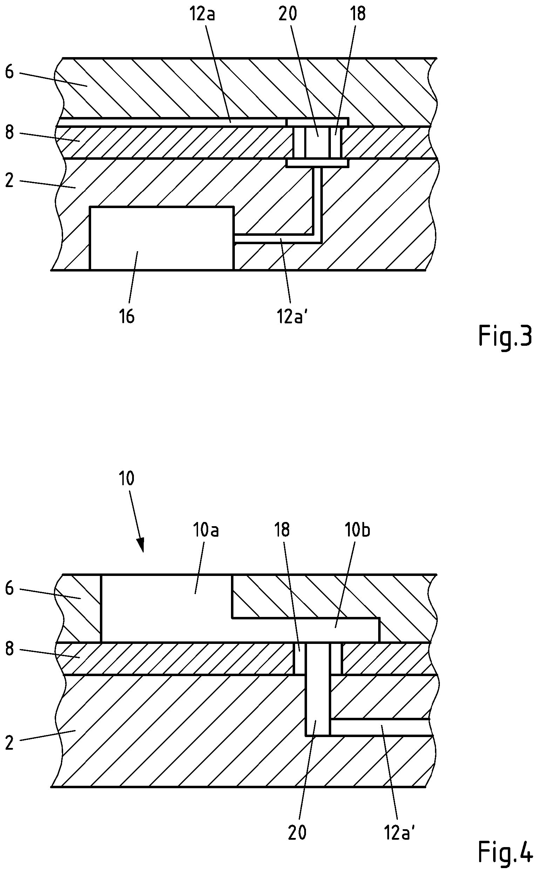

Fig. 3 zeigt eine weitere Möglichkeit der Kontaktierung von Leitern auf beiden Seiten der Barriereschicht 8. In der Fig. 3 ist die Barriereschicht 8 mit einer Durchbrechung 18 versehen. Die Durchbrechung 18 kann beispielsweise in der Form einer Stanzung, beispielweise rund oder eckig sein. Durch diese Durchbrechung 18 kann ein Kontaktelement 20 gesteckt werden, welches gegenüber der Barriereschicht 8 isoliert, ist, z.B. über einen Luftspalt. Das Kontaktelement 20 kann einerseits mit dem Leiter 12a und andererseits mit dem Leiter 12a' verbunden sein. Durch dieses Kontaktelement 20 erfolgt eine Kontaktierung der beiden Leiter 12a, a'. Gleiches oder alternatives kann natürlich auch für die Leiter 12b, b' erfolgen.

Fig. 4 zeigt ein weiteres Ausführungsbeispiel, bei dem unmittelbar im Bereich 10b des Sensors 10 eine Kontaktierung mit einem Kontaktelement 20 erfolgen kann. Hierbei kann der Sensor 10 mit seinem Bereich 10b über der Durchbrechung 18 angeordnet sein. Das Kontaktelement 20 kann unmittelbar mit einem Kontakt auf dem Sensor 10 verbunden werden. Ferner stellt das Kontaktelement 20 eine Verbindung mit einem Leiter 12a' her. Das Kontaktelement 20 kann Teil des Sensors 10 sein.

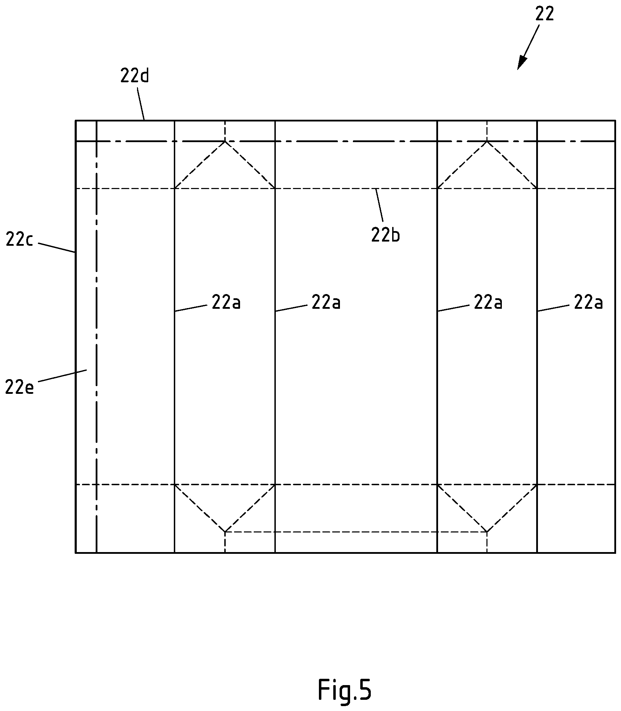

Fig. 5 zeigt eine Draufsicht auf einen Zuschnitt, beispielsweise von Seiten der Deckschicht 6. Der Zuschnitt 22 zeichnet sich durch Faltkanten 22a sowie in gestrichelten Linien dargestellten Falzungen 22b aus. Die Art eines Zuschnitts 22 ist hinlänglich bekannt Zu erkennen ist jedoch, dass an einer Längskante 22c oder an einer Querkante 22d ein mit strichpunktierten Linien gezeigter bevorzugter Bereich 22e ist, in dem die Trennstelle 14 oder die Durchbrechung 18 angeordnet ist Insbesondere im Bereich der Längskante 22c erfolgt ein Versiegeln der Verpackung mit der gegenüberliegenden Längskante, so dass dort eine Siegelnaht entsteht Im Bereich dieser Siegelnaht ist die Trennstelle 14 oder die Durchbrechung 18 bevorzugt angeordnet

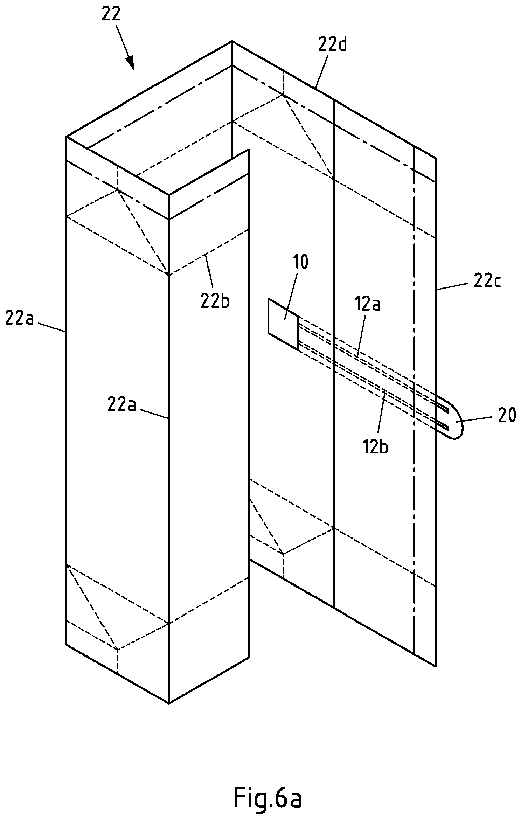

Auch ist es möglich, die Kontaktierung durch über eine Längskante 22c oder eine Querkante 22d hinausstehendes Element gebildet wird, wobei dieses Element nach dem Versiegeln umgebogen werden kann. Fig. 6a zeigt ein solches Beispiel. In der Fig. 6a ist ein vorgefalteter Packungsmantel dargestellt, bei der der Zuschnitt 22 bereits in großen Teilen vorgefaltet ist Ferner ist zu erkennen, dass ein Kontaktelement 20 mit den Leitern 12a, 12b einerseits mit einem Sensor 10 verbunden ist und andererseits über die Längskante 22c hinausragt. Dieses Kontaktelement 20 kann beispielsweise auf der Deckschicht 6 oder zwischen der Deckschicht 6 und der Barriereschicht 8 im Produktionsprozess vorgesehen werden.

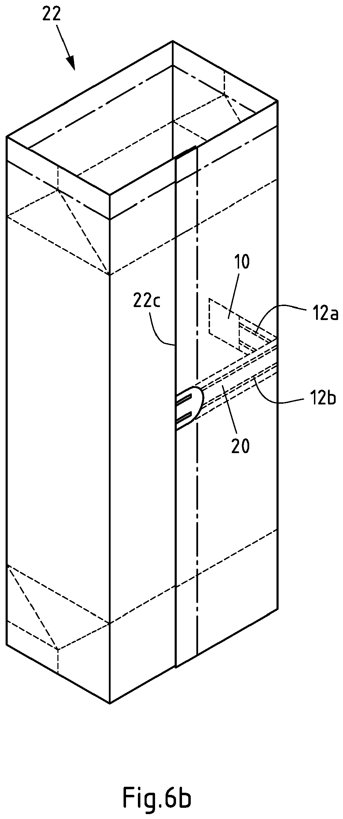

Anschließend erfolgt, wie in der Fig. 6b dargestellt ist, ein Verschließen der Verpackung entlang der Längskante 22c. Hierbei werden die Kanten bevorzugt miteinander versiegelt, so dass eine Deckschicht 6 mit einer Deckschicht 4 heiß gesiegelt wird. Das Kontaktelement 20 liegt dann nach dem Siegelvorgang einerseits im Inneren der Packung und andererseits außerhalb der Packung und ist durch die Siegelnaht hindurch durchgeführt. Abschließend kann der überstehende Teil um die Längskante 22c gelegt werden.

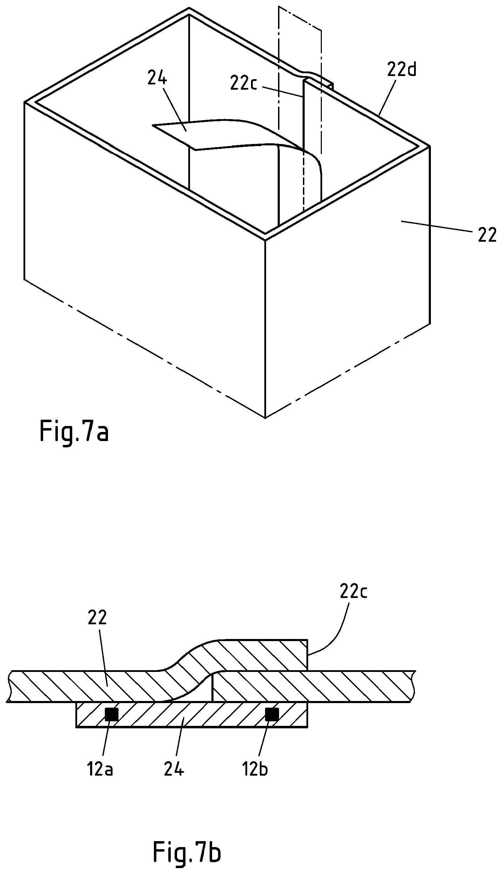

Auch ist es möglich, einen Siegelstreifen 24 mit den Leitungen 12a, b zu versehen. Fig. 7a zeigt ein weiteres Ausführungsbeispiel eines Packungsmantels, bei dem die Längskanten übereinander gelegt sind. In diesem Beispiel erfolgt ein Versiegeln des Zuschnitts 22 durch einen Siegelstreifen 24, der über die Nahtstelle gelegt wird. Der Siegelstreifen 24 verläuft dabei vorzugsweise entlang der gesamten Längsnaht 22c. Der Siegelstreifen 24 kann so gestaltet sein, dass er über die obere oder untere Kante hinaus absteht Nach dem Versiegeln ist der Siegelstreifen 24 an der Längskante angeordnet und steht, wie mit den gestrichelten Linien dargestellt, über die Querkante 22d des Packungsmantels hinaus. Nach dem Verschließen der Querkante kann der Siegelstreifen von außen kontaktiert werden und insbesondere die darin angeordneten Leitungen 12a, b. Ist keine Längsnaht vorhanden, z.B. bei einem Schlauch, kann ein als Streifen gebildetes Kontaktelement 20 auch, anstatt wie in Fig. 6b gezeigt, aus einer Querkante 22d herausragen. Dann kann das Kontaktelement 20 ohne Siegelfunktion versehen sein.

Fig. 7b zeigt einen Schnitt durch einen solchen Siegelstreifen 24. Zu erkennen ist, dass in dem Siegelstreifen 24 die Leiter 12a, b geführt sein können.

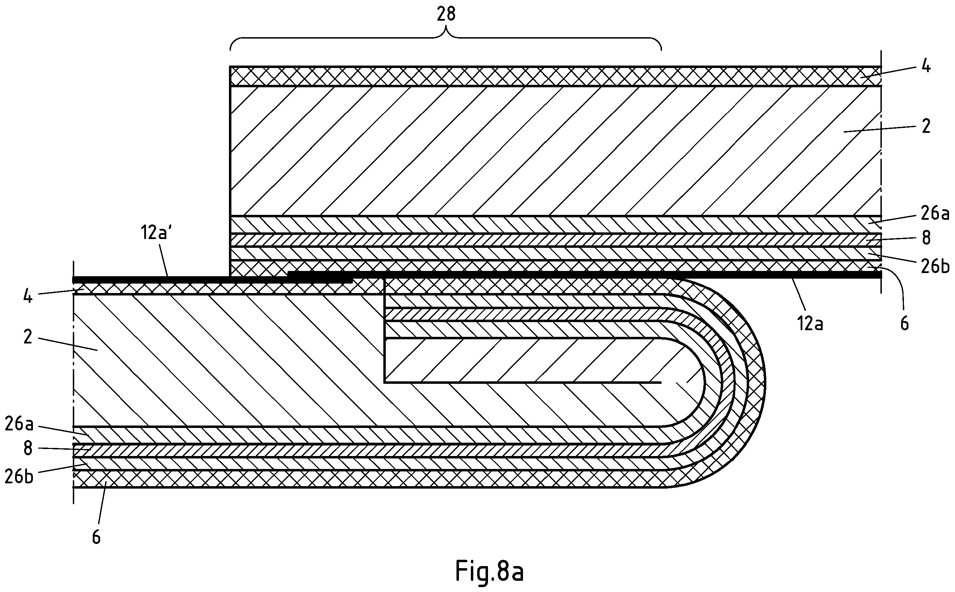

Die Fig. 8a-e zeigen ein Ausführungsbeispiel einer Versiegelung einer Längs- oder Querkante mit einer Schälung der Trägerschicht 2 und einem Umlegen einer Längskante 22c, wie dies aus dem Stand der Technik bekannt ist Die Kontaktierung von Leitern durch die Siegelnaht hindurch ist beispielhaft in den Fig. 8a-e dargestellt Die Fig. 8a-e beziehen sich jeweils auf einen Ausschnitt 30 im Bereich der Siegelnaht 28.

Fig. 8a zeigt einen Ausschnitt entlang einer Siegelnaht, beispielsweise entlang der Längskante 22c. Das Laminat ist aus der Trägerschicht 2, einer äußeren Deckschicht 4, einer inneren Deckschicht 6, einer Barriereschicht 8 sowie Hartvermittlerschichten 26a, b gebildet Fig. 8a zeigt, wie zwei gegenüberliegende Längskanten übereinander gelegt sind, wobei im Bereich einer Siegelnaht 28 eine Kante umgelegt ist Im Bereich der Siegelnaht 28 liegt somit die Deckschicht 6 einer ersten Längskante unmittelbar an der Deckschicht 6 der zweiten Längskante an. An dieser unmittelbaren Kontaktierung erfolgt eine Versiegelung der Deckschichten 6 miteinander.

Ferner ist in der Fig. 8a zu erkennen, dass im Bereich der Siegelnaht 28 die Trägerschicht 2 verjüngt ist, beispielsweise durch eine Schälung.

Fig. 8a zeigt beispielsweise einen Leiter 12a', der auf einer äußeren Deckschicht 4 aufliegt. An der inneren Deckschicht 6 liegt der Leiter 12a an. Im Bereich der Siegelnaht 28 überlappen sich der Leiter 12a und 12a' derart, dass ein elektrischer Kontakt gebildet werden kann. Ein so gebildeter elektrischer Kontakt ermöglicht es, eine elektrische Leitung zwischen einer Innenseite einer Verpackung und einer Außenseite einer Verpackung zu bilden. Durch die Siegelnaht 28 bleibt die Verpackung jedoch versiegelt.

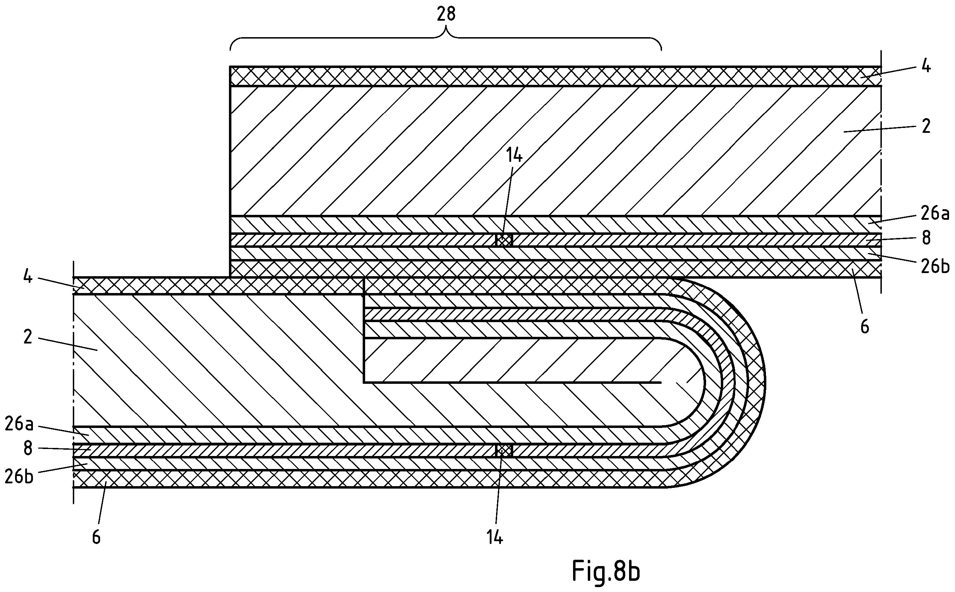

Fig. 8b zeigt ein weiteres Ausführungsbeispiel, bei dem die Trennstelle 14 in der Barriereschicht 8 ebenfalls im Bereich der Siegelnaht 28 angeordnet ist. Ist die Trennstelle 14 oder auch eine Durchbrechung 18 im Bereich der Siegelnaht 28, so hat diese keinerlei negativen Auswirkungen auf die Dichtigkeit der Barriereschicht 8. Diese liegt im Bereich der Siegelnaht 28 doppelt übereinander und die Siegelnaht 28 stellt somit eine äußerst Dichte Barriere dar. Auch kann kumulativ oder alternativ eine Trennstelle im Bereich der Barriereschicht 8 vorgesehen sein, welche dem Inneren der Verpackung, insbesondere dem Produkt zugewandt ist. Die Trennstelle 14 kann somit in dem Bereich der Barriereschicht 8 sein, in welchem die Schälung ist. Dabei kann die Trennstelle 8 in der äußeren Lage des Packungsmantels sein, der von einer inneren Lage abgedeckt ist. Alternativ oder kumulativ kann die Trennstelle 14 in einer inneren Lage des Packungsmantels sein, der unmittelbar im Kontakt mit dem Inneren der Packung insbesondere dem Produkt ist

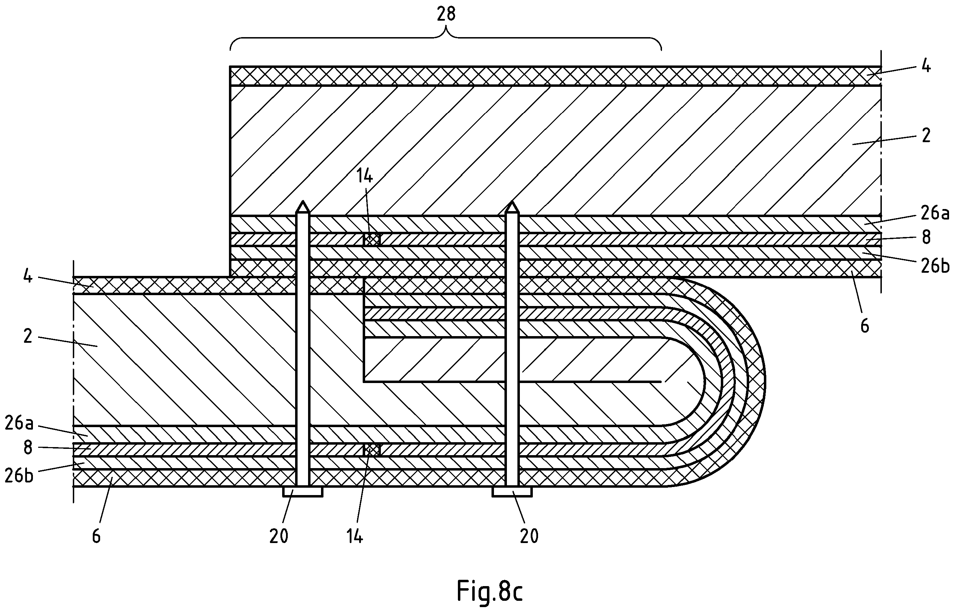

Fig. 8c zeigt ein weiteres Ausführungsbeispiel in dem ein Kontaktelement 20 einige der Schichten im Bereich der Siegelnaht durchbricht. Insbesondere durchbricht das Kontaktelement die beiden Barriereschichten 8, welche im Bereich der Siegelnaht 28 übereinander liegen. Auch können zwei nebeneinander angeordnete Kontaktelemente 20 die Barriereschicht 8 durchbrechen. Zwischen den Kontaktelementen 20 kann in jeder Lage des Packungsmantels jeweils in der Barriereschicht 8 eine Trennstelle vorgesehen sein.

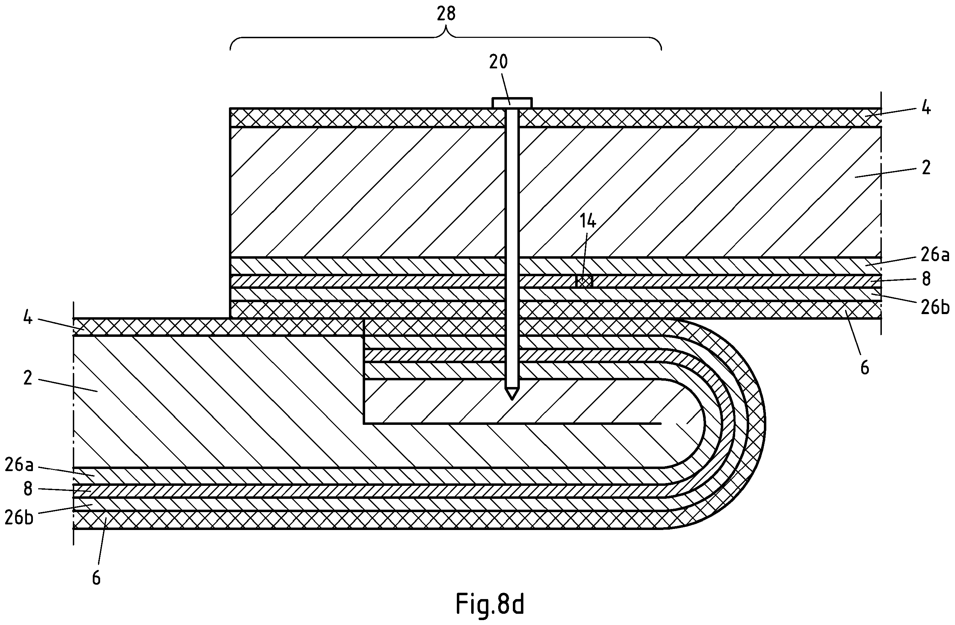

In den Anordnung in den Figuren 8c und d und auch unabhängig davon kann das Kontaktelement 20 auch teilweise isoliert sein. Insbesondere kann ein erstes Ende des Kontaktelement 20 mit einem Isolator beschichtet sein oder aus einem Isolator gebildet sein und ein zweites Ende kann auch eine elektrischen Leiter, z.B. Metall gebildet sein. Der Kopf des Kontaktelements 20 kann aus einem Metall oder einem Kunststoff gebildet sein. Das dem Kopf zugewandte Ende kann elektrisch isolierend sein. Die Spitze, also das dem Kopf abgewandte Ende kann elektrisch leitend sein.

Fig. 8d zeigt ein weiteres Ausführungsbeispiel, bei dem das Kontaktelement 20 mit seinem tellerförmigen Kopf von oben die Barriereschicht 8 durchbricht und die untere Barriereschicht 8 ebenfalls durchbricht

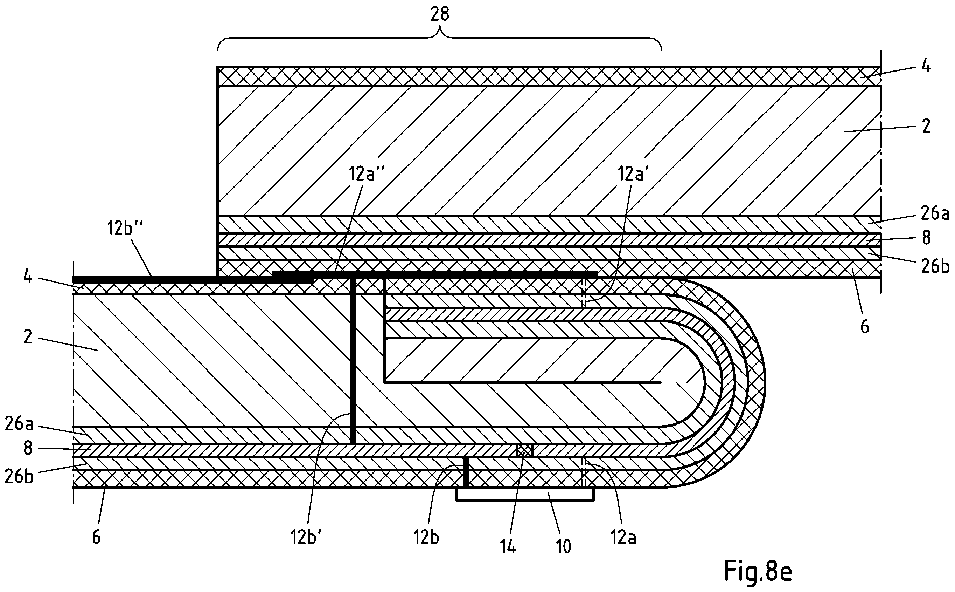

Fig. 8e zeigt ein weiteres Ausführungsbeispiel einer Kontaktierung eines Sensors 10, der auf einer inneren Deckschicht 6 angeordnet ist Hierzu weist der Sensor 10 Leitungen 12a, 12b auf, welche bis zur Barriereschicht 8 geführt sind. Die Barriereschicht 8 ist durch eine Trennstelle 14 in zwei Bereiche aufgeteilt und in jedem dieser Bereiche ist ein Leiter 12a', 12b' bis zu der Barriereschicht 8 geführt. Die beiden Leiter sind jeweils mit auf der inneren Deckschicht 8 angeordneten Leitern 12a", 12b" kontaktiert, welche ihrerseits wiederum mit auf der äußeren Deckschicht 6 angeordneten Leitern 12a''', 12b''' kontaktiert sind. Somit ist eine Durchkontaktierung im Bereich der Siegelnaht 28 von Inneren einer Verpackung zum Äußeren der Packung möglich.

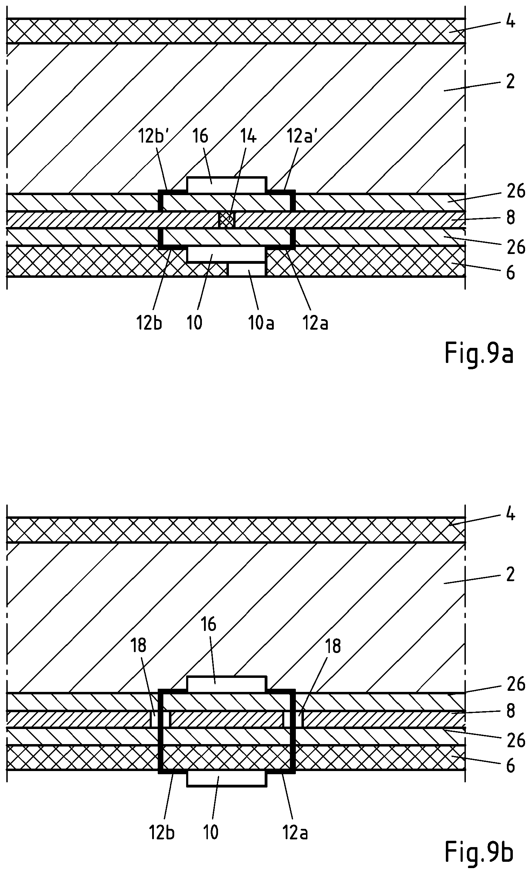

Fig. 9a-e zeigt erneut einen Schichtaufbau eines Packstoffs gebildet aus einer Deckschicht 4, einer Trägerschicht 2, einer Barriereschicht 8 und einer Deckschicht 6. Die Barriereschicht 8 ist jeweils von einer Haftvermittlerschicht 26 umgeben, die insbesondere als Isolator gebildet sein kann. Die Deckschicht 6 ist vorzugsweise dem Inneren einer Verpackung zugewandt, wohingegen die Deckschicht 4 dem Äußeren der Verpackung zugewandt ist

Fig. 9a zeigt eine Möglichkeit der Trennung der Barriereschicht 8 entlang einer Trennstelle 14. Ein Sensor 10 kann in einem Bereich 10a die innere Deckschicht 6 durchbrechen und somit eine unmittelbare Messung vornehmen. Es ist jedoch auch möglich, dass der Sensor 10 ausschließlich indirekte Messungen vornimmt und in keinem unmittelbaren Kontakt mit dem zu verpackenden Gut steht Ausgehend von dem Sensor 10 können auf der Haftvermittlerschicht 26 Leitungen 12a, b gebildet sein, die diese Schicht 26 durchbrechen und mit den jeweiligen Bereichen der Barriereschicht 8 verbunden sind. Auf der Außenseite der Packung können ebenfalls Leiter 12a, b mit einem Transmitter 16 verbunden sein, der auf der äußeren Haftvermittlerschicht 26 angeordnet ist und somit innerhalb der Trägerschicht 2 liegt.

Fig. 9b zeigt ein weiteres Ausführungsbeispiel, bei dem über Durchbrechungen 18, welche beispielsweise rund sein können, beispielsweise gestanzt sind, die Barriereschicht 8 durchbrochen ist. Durch diese Durchbrechungen sind die Leiter 12a, b isoliert geführt und kontaktieren den Sensor 10 mit dem Transmitter 16.

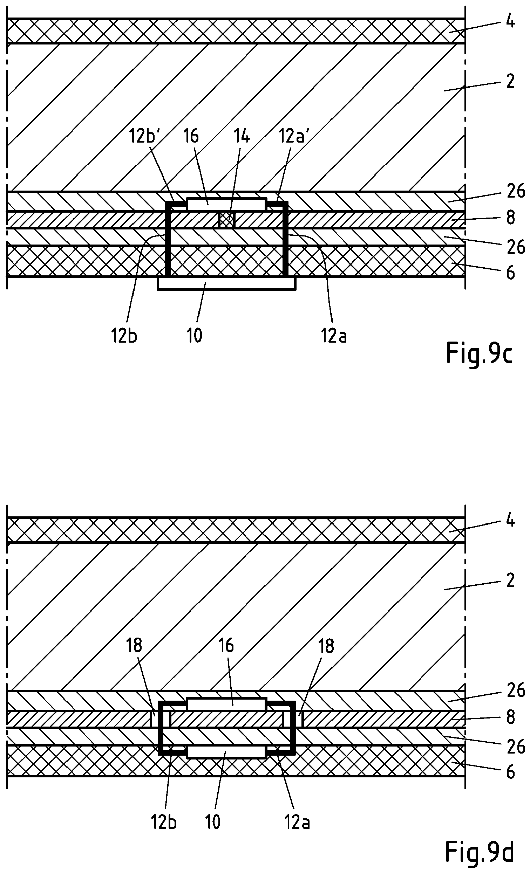

Fig. 9c zeigt ein weiteres Ausführungsbeispiel, bei dem der Sensor 10 auf der inneren Deckschicht 6 angeordnet ist Über eine Trennstelle 14 ist die Barriereschicht 8 zweigeteilt. Leiter 12a, b kontaktieren den Sensor 10 mit den beiden Teilen der Barriereschicht 8. Ein auf der Barriereschicht 8 aufgebrachter Transmitter 16, der gegenüber der Barriereschicht 8 isoliert sein kann, ist mit Leitern 12a', b' an den jeweiligen Bereichen der Barriereschicht 8 kontaktiert, so dass eine zweipolige Verbindung zwischen dem Sensor 10 und dem Transmitter 16 gebildet ist

Fig. 9d zeigt ein ähnliches Ausführungsbeispiel, wie Fig. 9c. Im Unterschied ist der Sensor 10 innerhalb der inneren Deckschicht 6 angeordnet und der Transmitter 16 liegt auf der Barriereschicht 8 auf.

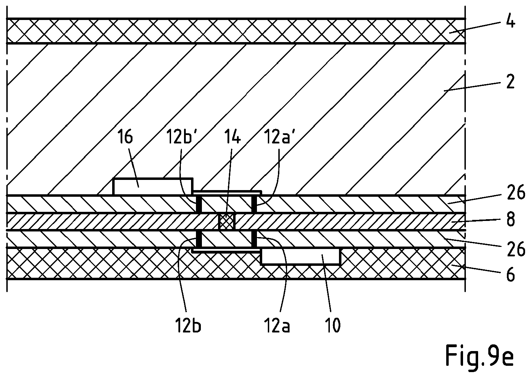

Fig. 9e zeigt ein weiteres Ausführungsbeispiel, bei dem eine Trennstelle 14 die Barriereschicht 8 in zwei Bereiche aufteilt Jeder Bereich ist mit jeweils einem Leiter 12a, b des Sensors 10 und einem Leiter 12a', b" des Transmitters 16 verbunden.

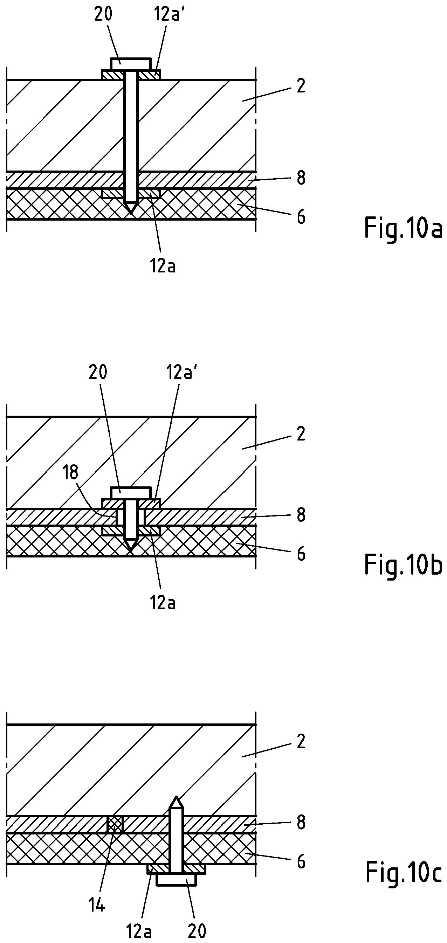

Fig. 10a zeigt ein Ausführungsbeispiel, bei dem ein Leiter 12a isoliert gegenüber der Barriereschicht 8 auf der Barriereschicht 8 aufliegt. Auf der Trägerschicht 2 liegt ein Leiter 12a' auf. Ein Kontaktelement 20 kann die Leiter 12a, a' und gleichzeitig die Trägerschicht 2 und die Barriereschicht 8 durchbrechen. Dabei ist das Kontaktelement 20 bevorzugt gegenüber der Barriereschicht 8 elektrisch isoliert.

Auch Fig. 10b zeigt ein Ausführungsbeispiel, bei dem die Leiter 12a, a' unmittelbar auf der Barriereschicht aufliegen und insbesondere hier gegenüber isoliert sind. Durch ein Kontaktelement 20, welches die Leiter 12a, a' durchbricht, können die einander überlappenden Leiter 12a, a' miteinander kontaktiert werden. Das Kontaktelement 20 ist bevorzugt gegenüber der Barriereschicht 8 isoliert oder in einer Durchbrechung 18 der Barriereschicht 8 geführt

Fig. 10c zeigt ein weiteres Ausführungsbeispiel, bei dem auf der inneren Deckschicht 6 der Leiter 12a beispielsweise aufgedruckt ist Diesen Leiter 12a durchsticht das Kontaktelement 20 und kontaktiert damit den überlappenden Leiter 12a. Ein Kontaktstift des Kontaktelements 20 durchbricht auch die Barriereschicht 8 und kontaktiert somit den Leiter 12a mit der Barriereschicht 8. In der Barriereschicht 8 ist eine Trennstelle 14 vorgesehen, so dass die Barriereschicht 8 in zwei Bereiche unterteilt ist In dem jeweils anderen Bereich kann eine ähnliche Kontaktierung mit einem Leiter 12b und einem weiteren Kontaktelement 20 erfolgen. Das Kontaktelement 20 kann bis in die Trägerschicht 2 hineinragen.

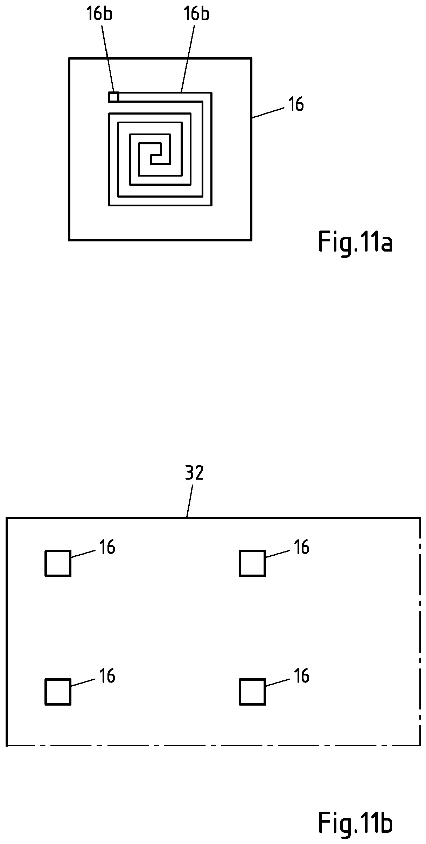

Fig. 11a zeigt ein Transmitter 16. Zu erkennen ist, dass der Transmitter 16 eine Antennenstruktur 16a sowie einen Prozessor 16b und gegebenenfalls einen Speicher aufweist

Ein solcher Transmitter 16 kann beispielsweise auf einer Folienbahn 32 aufgebracht sein und in definierten Abständen darauf angeordnet sein. Eine solche Folienbahn 32 kann im Herstellungsprozess verwendet werden, um für jeweils einen Zuschnitt einen Transmitter 16 auf die Trägerschicht 2 oder die Deckschicht 4 aufzubringen. Das gleiche gilt natürlich auch für einen Sensor 10, der in ähnlicher Art und Weise auf die Deckschicht 6 oder die Barriereschicht 8 aufgebracht werden kann.

### Bezugszeichenliste

- 2: Trägerschicht
- 4: Deckschicht
- 6: Deckschicht
- 8: Barriereschicht
- 10: Sensor
- 10a, b: Bereich
- 12a, b: Leiter
- 14: Trennstelle
- 16: Transmitter
- 18: Durchbrechung
- 20: Kontaktelement
- 22: Zuschnitt
- 22a: Faltkante
- 22b: Falzung
- 22c: Längskante
- 22d: Querkante
- 22e: Bereich
- 24: Siegelstreifen
- 26: Haftvermittlerschicht
- 28: Siegelnaht
- 30: Ausschnitt
- 32: Folienbahn

## Patentansprüche

1. Zuschnitt aus einem Packungslaminat zur Bildung einer Packung für fließfähige Produkte mit
- zumindest einer Trägerschicht,
- einer elektrisch leitenden Barriereschicht und
- einer Deckschicht, wobei
- auf einer ersten Seite der Barriereschicht ein Funktionselement angeordnet ist und
- auf der dem Funktionselement abgewandten zweiten Seite der Barriereschicht ein elektrisches Element angeordnet ist, wobei
- das Funktionselement mit dem elektrischen Element über zumindest zwei zueinander isolierte elektrische Leiter verbunden ist,
**dadurch gekennzeichnet,**
- **dass** die erste Seite der Barriereschicht einer Innenschicht einer Packung zugewandt ist und dass die zweite Seite der Barriereschicht einer Außenschicht der Packung zugewandt ist, und
- **dass** zumindest einer der Leiter um eine Schnittkante des Zuschnitts gelegt ist, so dass ein Teil des Leiters auf der ersten Seite der Barriereschicht angeordnet ist und ein Teil des Leiters auf der zweiten Seite der Barriereschicht

2. Zuschnitt nach Anspruch 1,
**dadurch gekennzeichnet,**
- **dass** das Funktionselement einen Sensor aufweist und/oder dass das elektrische Element ein Transmitter und/oder eine Antenne aufweist.

3. Zuschnitt nach Anspruch 1 oder 2,
**dadurch gekennzeichnet,**
- **dass** die Barriereschicht zumindest einen Teil eines der elektrischen Leiter bildet.

4. Zuschnitt nach einem der vorangehenden Ansprüche,
**dadurch gekennzeichnet,**
- **dass** die Barriereschicht eine Ausnehmung aufweist und dass zumindest einer der elektrischen Leiter durch die Ausnehmung geführt ist

5. Zuschnitt nach einem der vorangehenden Ansprüche,
**dadurch gekennzeichnet,**
- **dass** das Funktionselement mit einer Schicht auf der ersten Seite der Barriereschicht verbunden ist und/oder dass das elektrische Element mit einer Schicht auf der zweiten Seite der Barriereschicht verbunden ist

6. Zuschnitt nach einem der vorangehenden Ansprüche,
**dadurch gekennzeichnet,**
- **dass** die Barriereschicht zumindest entlang einer Richtung durchgehend in zwei voneinander getrennte Bereiche aufgetrennt ist und dass ein erster Bereich zumindest einen Teil des ersten elektrischen Leiters bildet oder dass ein zweiter Bereich zumindest einen Teil des zweiten elektrischen Leiters bildet

7. Zuschnitt nach einem der vorangehenden Ansprüche,
**dadurch gekennzeichnet,**
- **dass** auf einer Schicht auf der ersten Seite der Barriereschicht und einer Schicht auf der zweiten Seite der Barriereschicht jeweils zumindest ein Teil eines der elektrischen Leiter aufgebracht ist, wobei die jeweiligen Teile sich einander teilweise überlappen.

8. Zuschnitt nach einem der vorangehenden Ansprüche,
**dadurch gekennzeichnet,**
- **dass** auf der ersten Seite der Barriereschicht zumindest eine Deckschicht aufgebracht ist und dass das Funktionselement und/oder einer der elektrischen Leiter zwischen der Barriereschicht und der Deckschicht oder auf der der Barriereschicht abgewandten Seite der Deckschicht aufgebracht oder eingebracht ist und/oder
- **dass** auf der zweiten Seite der Barriereschicht zumindest eine Trägerschicht aufgebracht ist und dass das elektrische Element und/oder einer der elektrischen Leiter zwischen der Barriereschicht und der Trägerschicht oder auf einer Schicht die auf der Barriereschicht abgewandten Seite der Trägerschicht angeordnet ist, aufgebracht oder eingebracht ist

9. Zuschnitt nach einem der vorangehenden Ansprüche,
**dadurch gekennzeichnet,**
- **dass** zumindest einer der Leiter winklig, insbesondere rechtwinklig zu einer Schnittkante des Zuschnitts verläuft, insbesondere
- **dass** zumindest einer der Leiter auf einer auf der ersten Seite der Barriereschicht angeordneten Deckschicht im Bereich einer Schnittkante des Zuschnitts verläuft.

10. Zuschnitt nach einem der vorangehenden Ansprüche,
**dadurch gekennzeichnet,**
- **dass** ein festes Ende zumindest eines Leiters auf der ersten Seite der Barriereschicht angeordnet befestigt ist und dass ein freies Ende des zumindest einen Leiters über eine Schnittkante hinaus ragt.

11. Zuschnitt nach einem der vorangehenden Ansprüche,
**dadurch gekennzeichnet,**
- **dass** ein freies Ende des Leiters über eine Längskante oder eine Querkante hinaus ragt.

12. Zuschnitt nach einem der vorangehenden Ansprüche,
**dadurch gekennzeichnet,**
- **dass** die Barriereschicht zumindest entlang einer Richtung durchgehend in zwei voneinander getrennte Bereiche aufgetrennt ist, wobei die Trennung zwischen den Bereichen im Bereich einer Längskante oder einer Querkante ist, insbesondere im Bereich einer Schälung der Kante oder einer Siegelnaht der Kante und/oder
- **dass** die Barriereschicht eine Ausnehmung aufweist und dass zumindest einer der elektrischen Leiter durch die Ausnehmung geführt ist, wobei die Ausnehmung im Bereich einer Längskante oder einer Querkante ist, insbesondere im Bereich einer Schälung der Längskante und/oder der Querkante, vorzugsweise im Bereich einer Siegelnaht der Längskante und/oder der Querkante.

13. Packungsmantel aus einem Zuschnitt nach einem der vorangehenden Ansprüche.

14. Packungsmantel nach einem der vorangehenden Ansprüche,
**dadurch gekennzeichnet,**
- **dass** im Bereich der ersten Seite der Barriereschicht eine siegelfähige Deckschicht vorgesehen ist, dass die siegelfähige Deckschicht im Bereich zumindest einer Naht gesiegelt ist, wobei zumindest einer der Leiter im Bereich der Siegelnaht verläuft und/oder
- **dass** eine Naht entlang einer Längskante oder einer Querkante mit einem Siegelstreifen versiegelt ist und dass zumindest einer der Leiter in dem Siegelstreifen angeordnet ist.

15. Packungsmantel nach einem der vorangehenden Ansprüche,
**dadurch gekennzeichnet,**
- **dass** die Trennung der Barriereschicht zwischen den zwei Bereichen im Bereich der Siegelnaht angeordnet ist und/oder
- **dass** die Ausnehmung in der Barriereschicht im Bereich der Siegelnaht angeordnet ist.

16. Packung aus einem Packungsmantel nach einem der Ansprüche 13 bis 2215

17. Verpackung aus einer Packung nach Anspruch 16.

## Claims

1. Blank from a package laminate for forming a package for flowable products with
- at least one carrier layer,
- an electrically conductive barrier layer and
- a top layer, wherein
- a functional element is arranged on a first side of the barrier layer, and
- an electrical element is arranged on the second side of the barrier layer facing away from the functional element, wherein
- the functional element is connected to the electrical element via at least two mutually insulated electrical conductors
**characterized in that**,
- the first side of the barrier layer faces an inner layer of a package, and **in that** the second side of the barrier layer faces an outer layer of a package and
- at least one of the conductors is laid around a cut edge of the blank so that a portion of the conductor is disposed on the first side of the barrier layer and a portion of the conductor is disposed on the second side of the barrier layer.

2. Blank according to claim 1,
**characterized in that**
- the functional element has a sensor and/or **in that** the electrical element has a transmitter and/or an antenna.

3. Blank according to claim 1 or 2,
**characterized in that**
- the barrier layer forms at least part of one of the electrical conductors.

4. Blank according to one of the preceding claims,
**characterized in that**
- the barrier layer has a recess, and **in that** at least one of the electrical conductors is passed through the recess.

5. Blank according to one of the preceding claims,
**characterized in that**
- the functional element is connected to a layer on the first side of the barrier layer and/or **in that** the electrical element is connected to a layer on the second side of the barrier layer.

6. Blank according to one of the preceding claims,
**characterized in that**
- the barrier layer is continuously separated into two regions which are separated from one another along at least one direction, and **in that** a first region forms at least part of the first electrical conductor and/or **in that** a second region forms at least part of the second electrical conductor.

7. Blank according to one of the preceding claims,
**characterized in that**
- at least part of one of the electrical conductors is in each case applied to a layer on the first side of the barrier layer and a layer on the second side of the barrier layer, the respective parts partially overlapping one another.

8. Blank according to one of the preceding claims,
**characterized in that**
- at least one covering layer is applied to the first side of the barrier layer, and **in that** the functional element and/or one of the electrical conductors is applied or introduced between the barrier layer and the covering layer or on the side of the covering layer facing away from the barrier layer and/or
- at least one carrier layer is applied to the second side of the barrier layer and **in that** the electrical element and/or one of the electrical conductors is applied or introduced between the barrier layer and the carrier layer or on a layer which is arranged on the side of the carrier layer facing away from the barrier layer.

9. Blank according to one of the preceding claims,
**characterized in that**
- at least one of the conductors extends at an angle, in particular at right angles to a cut edge of the blank and/or
- at least one of the conductors extends on a top layer arranged on the first side of the barrier layer in the region of a cut edge of the blank.

10. Blank according to one of the preceding claims,
**characterized in that**
- a fixed end of at least one conductor is fixed arranged on the first side of the barrier layer, and **in that** a free end of the at least one conductor projects beyond a blank edge.

11. Blank according to one of the preceding claims,
**characterized in that**
- the free end of the conductor projects beyond a longitudinal edge or a transverse edge.

12. Blank according to one of the preceding claims,
**characterized in that**
- the barrier layer is continuously separated into two regions which are separated from one another along at least one direction, wherein the separation between the regions being in the region of a longitudinal edge or a transverse edge, in particular in the region of a peeling of the edge or a sealed seam of the edge and/or
- the barrier layer has a recess and **in that** at least one of the electrical conductors is passed through the recess, the recess being in the region of a longitudinal edge or a transverse edge, in particular in the region of a peeling of the longitudinal edge and/or the transverse edge, preferably in the region of a sealed seam of the longitudinal edge and/or the transverse edge.

13. Sleeve made of a blank according to one of the preceding claims.

14. Sleeve according to claim 13,
**characterized in that**
- a sealable top layer is provided in the region of the first side of the barrier layer, **in that** the sealable top layer is sealed in the region of at least one seam, at least one of the conductors running in the region of the sealed seam and/or
- a seam along a longitudinal edge or a transverse edge is sealed with a sealing strip, and **in that** at least one of the conductors is arranged in the sealing strip.

15. Sleeve according to one of the preceding claims,
**characterized in that**
- the separation of the barrier layer between the two areas is arranged in the area of the sealing seam and/or
- the recess in the barrier layer is arranged in the area of the sealing seam.

16. Package made of a sleeve according to any of claims 13 to 15.

17. Packaging made of a package according to claim 16.

## Revendications

1. Une pièce découpée d'un stratifié d'emballage pour former un emballage pour des produits fluides avec
- au moins une couche support
- une couche barrière électriquement conductrice et
- une couche supérieure, où
- un élément fonctionnel est disposé sur un premier côté de la couche barrière et
- un élément électrique est disposé sur le second côté de la couche barrière, qui est opposé de l'élément fonctionnel, où
- l'élément fonctionnel est relié à l'élément électrique par au moins deux conducteurs électriques isolés l'un de l'autre,
**caractérisé en ce**
- **que** le premier côté de la couche barrière fait face à une couche intérieure d'un emballage et que le second côté de la couche barrière fait face à une couche extérieure de l'emballage, et
- **qu'**au moins l'un des conducteurs est disposé autour d'un bord découpé de la pièce découpée de telle sorte qu'une partie du conducteur est disposée sur le premier côté de la couche barrière et une partie du conducteur soit disposée sur le second côté de la couche barrière.

2. Pièce découpée selon la revendication 1,
**caractérisé en ce**
- **que** l'élément fonctionnel comprend un capteur et/ou que l'élément électrique comprend un transmetteur et/ou une antenne.

3. Pièce découpée selon la revendication 1 ou 2,
**caractérisé en ce**
- **que** la couche barrière forme au moins une partie de l'un des conducteurs électriques.

4. Pièce découpée selon l'une des revendications précédentes,
**caractérisé en ce**
- **que** la couche barrière comprend un orifice et qu'au moins un des conducteurs électriques est guidé à travers l'orifice.

5. Pièce découpée selon l'une des revendications précédentes,
**caractérisé en ce**
- **que** l'élément fonctionnel est connecté à une couche sur le premier côté de la couche barrière et/ou que l'élément électrique est connecté à une couche sur le second côté de la couche barrière.

6. Pièce découpée selon l'une quelconque des revendications précédentes, **caractérisé par**
- que la couche barrière est séparée entièrement en deux régions séparées l'une de l'autre au moins le long d'une direction et qu'une première région forme au moins une partie du premier conducteur électrique ou qu'une deuxième région forme au moins une partie du deuxième conducteur électrique.

7. Pièce découpée selon l'une des revendications précédentes,
**caractérisé en ce**
- **qu'**à une couche sur le premier côté de la couche barrière et à une couche sur le second côté de la couche barrière au moins une partie de l'un des conducteurs électriques est appliquée, respectivement, où les parties respectives se recouvrant partiellement l'une l'autre.

8. Pièce découpée selon l'une des revendications précédentes,
**caractérisé en ce**
- **qu'**au moins une couche supérieure est appliquée sur le premier côté de la couche barrière, et que l'élément fonctionnel et/ou l'un des conducteurs électriques est appliqué ou inséré entre la couche barrière et la couche supérieure ou sur le côté de la couche supérieure opposé à la couche barrière, et/ou
- **qu'**au moins une couche support est appliquée sur la deuxième côté de la couche barrière, et que l'élément électrique et/ou l'un des conducteurs électriques est appliqué ou incorporé entre la couche barrière et la couche support ou sur une couche qui est disposée sur la face de la couche support opposée à la couche barrière.

9. Pièce découpée selon l'une des revendications précédentes,
**caractérisé en ce**
- **qu'**au moins l'un des conducteurs fait un angle, en particulier un angle droit, avec un bord découpé de la pièce découpée, en particulier
- **qu'**au moins l'un des conducteurs passe sur une couche supérieur disposée sur le premier côté de la couche barrière dans la région d'un bord découpée de la pièce découpée.

10. Pièce découpée selon l'une des revendications précédentes,
**caractérisé en ce**
- **qu'**une extrémité fixe d'au moins un conducteur est disposée de manière fixe sur le premier côté de la couche barrière et qu'une extrémité libre de l'au moins un conducteur dépasse d'un bord de coupe.

11. Pièce découpée selon l'une quelconque des revendications précédentes, **caractérisé par**
- qu'une extrémité libre du conducteur dépasse un bord longitudinal ou un bord transversal.

12. Pièce découpée selon l'une des revendications précédentes,
**caractérisé en ce**
- **que** la couche barrière est séparée de manière continue au moins le long d'une direction en deux régions séparées l'une de l'autre, où la séparation entre les régions est dans la région d'un bord longitudinal ou d'un bord transversal, en particulier dans la région d'une pelure du bord ou d'un joint scellé du bord, et/ou
- **que** la couche barrière comprend un orifice et qu'au moins un des conducteurs électriques est guidé à travers l'orifice, où l'orifice se trouve dans la région d'un bord longitudinal ou d'un bord transversal, en particulier dans la région d'une pelure du bord longitudinal et/ou du bord transversal, de préférence dans la région d'un joint scellé du bord longitudinal et/ou du bord transversal.

13. Enveloppe d'emballage réalisée à partir d'une pièce découpée selon l'une des revendications précédentes.

14. Enveloppe d'emballage selon l'une des revendications précédentes,
**caractérisé en ce**
- **qu'**une couche supérieure scellable est disposée dans la région du premier côté de la couche barrière, que la couche supérieur scellable est scellée dans la région d'au moins une couture, où au moins un des conducteurs passe dans la région de la couture scellée et/ou
- **qu'**une couture est scellée le long d'un bord longitudinal ou d'un bord transversal avec une bande d'étanchéité, et qu'au moins l'un des conducteurs est disposé dans la bande d'étanchéité.

15. Enveloppe d'emballage selon l'une des revendications précédentes,
**caractérisé en ce**
- **que** la séparation de la couche barrière entre les deux régions est arrangée dans la région du joint scellé et/ou
- **que** l'orifice dans la couche barrière est arrangé dans la région du joint scellé.

16. Paquet réalisé à partir d'une enveloppe d'emballage selon l'une des revendications 13 à 15.

17. Emballage réalisé à partir d'un paquet selon la revendication 16.
